# EUROPEAN PATENT SPECIFICATION

(11) **EP 1 660 135 B1**
(45) Date of publication and mention of the grant of the patent: **03.06.2009**
(21) Application number: 04777732.1
(22) Date of filing: 09.07.2004
(51) Int. Cl.: A61K 48/00, A61K 39/395

(54) **METHOD OF TREATING HEPATOCELLULAR CARCINOMA**
VERFAHREN ZUR BEHANDLUNG VON HEPATOZELLULÄREM KARZINOM
PROCEDE DE TRAITEMENT DE CARCINOME HEPATOCELLULAIRE

(30) Priority: 25.07.2003 US 490047 P
(43) Date of publication of application: 31.05.2006
(73) Proprietor: ZymoGenetics, Inc., Seattle, WA 98102 (US)
(72) Inventor: PALMER, Thomas, E., Bellevue, WA 98008 (US)
(74) Representative: Bizley, Richard Edward
(86) International application number: PCT/US2004/021835
(87) International publication number: WO 2005/011742

(56) References cited:
- US-A1- 2003 087 870
- US-B1- 6 432 673
- LI XURI ET AL: "PDGF-C is a new protease-activated ligand for the PDGF alpha-receptor" NATURE CELL BIOLOGY, vol. 2, no. 5, May 2000 (2000-05), pages 302-309, XP002303789 ISSN: 1465-7392

## Description

### BACKGROUND OF THE INVENTION

Hepatocellular carcinoma, or hepatoma, is the most common type of primary liver cancer. It is particularly common (and an important cause of death) in parts of Africa and southeast Asia where chronic hepatitis B is endemic.

The precise etiology of hepatocellular carcinoma (HCC) is unknown. The tumors are composed of malignant hepatocytes. There is a strong correlation with hepatitis B incidence, and incorporation of viral DNA into the host genome is believed to lead to malignant transformation. Chronic hepatitis C infection has also been linked to HCC, although the hepatitis C virus (an RNA virus) does not incorporate into the host genome. Fibrogenesis (cirrhosis) in hepatitis C patients may play a role in tumor formation. HCC is also a major complication of hemochromatosis. Other risk factors for HCC include environmental toxins (e.g., aflatoxins), alcoholism, family history, and being male.

Signs and symptoms of HCC include pain in the right upper abdomen, presence of tissue mass or swollen abdomen, weight loss, loss of appetite and feelings of fullness, weekness or tiredness, nausea and vomiting, jaundice, and fever. Serum α-fetoprotein and des-g-carboxy-prothrombin are diagnostic markers. Additional diagnostic methods include ultrasound, CT scanning, magnetic resonance imaging, hepatic arteriography, and biopsy.

Current treatments for HCC have achieved only limited success. Surgery is most satisfactory, and can result in prolonged survival of patients with small, localized tumors. Treatment of more advanced HCC includes chemotherapy and/or radiation therapy. Liver transplantation has also been used with some long-term success. However, the success of these treatments is largely dependent upon early diagnosis. Other reported treatments for HCC include radiofrequency ablation (Livraghi et al., Radiology 210:655-61, 1999; Curley et al., Ann Surg 230:1-8, 1999); laser or microwave therapy; percutaneous ethanol injection (Livraghi et al. ibid.; Tanaka et al., Cancer 82:78-85, 1998); cryosurgery (Zhou and Tang, Semin. Surg. Oncol. 14:171-174, 1998); hepatic arterial infusion with, for example, ¹²⁵I-lipiodol (Lau et al., Lancet 353(9155):797-801, 1999), 5-fluorodeoxyuridine or dichloromethotrexate (Ensminger et al., Cancer Treat Rep. 65:393-400, 1981); chemoembolization using, for example, bischlorethymitrosourea (Dakhil et al., Cancer 50:631-635, 1982) or iodized oil and doxorubicin hydrochloride (Choi et al, Radiology 182:709-713, 1992); immunotherapy (Takayama, et al., Lancet 356(9232):802-807, 2000); and a combination of cisplatin chemotherapy with radiation (Epstein et al., Cancer 67:896-900, 1991).

### DESCRIPTION OF THE INVENTION

The present invention provides materials and methods for treating hepatocellular carcinoma in a mammal.

Within one aspect of the present disclosure there is provided a method of treating hepatocellular carcinoma in a mammal comprising administering to a mammal having a hepatocellular carcinoma a composition comprising a zvegf3 antagonist in combination with a pharmaceutically acceptable delivery vehicle, wherein the zvegf3 antagonist is selected from the group consisting of anti-zvegf3 antibodies, mitogenically inactive receptor-binding zvegf3 variant polypeptides, and inhibitory polynucleotides, in an amount sufficient to produce a tumor response in the mammal. Within one embodiment, a tumor response is measured as a complete response, a partial response, or a reduction in time to progression. Within another embodiment, the zvegf3 antagonist is selected from the group consisting of anti-zvegf3 antibodies and inhibitory polynucleotides. Within a further embodiment, the antagonist is an anti-zvegf3 antibody. Within a related embodiment, the antibody is a monoclonal antibody, such as an IgG monoclonal antibody. Within other embodiments of the invention, the zvegf3 antagonist is an antibody that specifically binds to a dimeric protein having two polypeptide chains, wherein each of the polypeptide chains consists of a sequence of amino acid residues selected from the group consisting of residues 230-345 of SEQ ID NO:2, residues 231-345 of SEQ ID NO:2, residues 232-345 of SEQ ID NO:2, residues 233-345 of SEQ ID NO:2, residues 234-345 of SEQ ID NO:2, residues 235-345 of SEQ ID NO:2, residues 236-345 of SEQ ID NO:2, residues 237-345 of SEQ ID NO:2, residues 238-345 of SEQ ID NO:2, residues 239-345 of SEQ ID NO:2, and residues 240-345 of SEQ ID NO:2. Within an additional embodiment, the zvegf3 antagonist is administered by intravenous infusion.

Within a second aspect of the disclosure there is provided a method of reducing cancer cell proliferation comprising administering to a mammal with hepatocellular carcinoma an amount of a composition comprising a zvegf3 antagonist in combination with a pharmaceutically acceptable delivery vehicle, wherein the zvegf3 antagonist is selected from the group consisting of anti-zvegf3 antibodies, mitogenically inactive receptor-binding zvegf3 variant polypeptides, and inhibitory polynucleotides, in an amount sufficient to reduce cancer cell proliferation within the hepatocellular carcinoma. Within one embodiment, the zvegf3 antagonist is selected from the group consisting of anti-zvegf3 antibodies and inhibitory polynucleotides. Within a further embodiment, the antagonist is an anti-zvegf3 antibody. Within a related embodiment, the antibody is a monoclonal antibody, such as an IgG monoclonal antibody. Within other embodiments of the invention, the zvegf3 antagonist is an antibody that specifically binds to a dimeric protein having two polypeptide chains, wherein each of the polypeptide chains consists of a sequence of amino acid residues selected from the group consisting of residues 230-345 of SEQ ID NO:2, residues 231-345 of SEQ ID NO:2, residues 232-345 of SEQ ID NO:2, residues 233-345 of SEQ ID NO:2, residues 234-345 of SEQ ID NO:2, residues 235-345 of SEQ ID NO:2, residues 236-345 of SEQ ID NO:2, residues 237-345 of SEQ ID NO:2, residues 238-345 of SEQ ID NO:2, residues 239-345 of SEQ ID NO:2, and residues 240-345 of SEQ ID NO:2. Within an additional embodiment, the zvegf3 antagonist is administered by intravenous infusion.

Within a third aspect of the disclosure there is provided the use of a zvegf3 antagonist in the preparation of a medicament for treatment of hepatocellular carcinoma in a mammal, wherein the zvegf3 antagonist is selected from the group consisting of anti-zvegf3 antibodies, mitogenically inactive receptor-binding zvegf3 variant polypeptides, and inhibitory polynucleotides. Within one embodiment the medicament is formulated for intravenous infusion.

These and other aspects of the invention will become evident upon reference to the following detailed description of the invention and the attached drawings. In the drawings:
Figs. 1A - 1G are a Hopp/Woods hydrophilicity profile of the amino acid sequence shown in SEQ ID NO:2. The profile is based on a sliding six-residue window. Buried G, S, and T residues and exposed H, Y, and W residues were ignored. These residues are indicated in the figure by lower case letters.
Fig. 2 is an alignment of human (SEQ ID NO:2) and mouse (SEQ ID NO:4) amino acid sequences.

The term "antagonist" is used herein to denote a compound that reduces a biological activity of another compound. Within the present invention, a "zvegf3 antagonist" is a compound that reduces the receptor-mediated biological activity (e.g., mitogenic activity) of zvegf3 on a target cell. Antagonists may exert their action by competing with zvegf3 for binding sites on a cell-surface receptor, by binding to zvegf3 and preventing it from binding to a cell-surface receptor, by otherwise interfering with receptor function, by reducing production of zvegf3, or by other means.

The term "cancer" or "cancer cell" is used herein to denote a tissue or cell found in a neoplasm which possesses characteristics which differentiate it from normal tissue or tissue cells. Such characteristics include but are not limited to degree of anaplasia, irregularity in shape, indistinctness of cell outline, nuclear size, changes in structure of nucleus or cytoplasm, other phenotypic changes, presence of cellular proteins indicative of a cancerous or pre-cancerous state, increased number of mitoses, and ability to metastasize. Words pertaining to "cancer" include carcinoma, sarcoma, tumor, epithelioma, adenoma, leukemia, lymphoma, polyp, scirrus, transformation, neoplasm, and the like.

An "inhibitory polynucleotide" is a DNA or RNA molecule that reduces or prevents expression (transcription or translation) of a second (target) polynucleotide. Inhibitory polynucleotides include antisense polynucleotides, ribozymes, and external guide sequences. The term "inhibitory polynucleotide" further includes DNA and RNA molecules that encode the actual inhibitory species, such as DNA molecules that encode ribozymes.

The term "neoplastic", when referring to cells, indicates cells undergoing new and abnormal proliferation, particularly in a tissue wherein the proliferation is uncontrolled and progressive, resulting in a neoplasm. The neoplastic cells can be either malignant, i.e. invasive and metastatic, or benign.

The terms "treat" and "treatment" are used broadly to denote therapeutic and prophylactic interventions that favorably alter a pathological state. Treatments include procedures that moderate or reverse the progression of, reduce the severity of, prevent, or cure a disease. In the case of cancers, treatment includes an increase in survival rate over a given time period or an increase in survival time, reduction in tumor mass, reduction in tumor metastasis, cessation of disease progression, reduction in time to progression, and the like.

The present disclosure provides methods for treating hepatocellular carcinoma in a patient using zvegf3 antagonists. Zvegf3 is a protein that is structurally related to platelet-derived growth factor (PDGF) and the vascular endothelial growth factors (VEGF). This protein has also been designated "VEGF-R" (WIPO Publication WO 99/37671) and, more recently, "PDGF-C" (WO 00/18212; Li et al., Nature Cell Biol. 2:302-309, 2000; Cao et al., FASEB J. 16:1575-1583, 2002). Zvegf3/PDGF-C is a multi-domain protein with significant homology to the PDGF/VEGF family of growth factors. Representative amino acid sequences of human and mouse zvegf3 are shown in SEQ ID NO:2 and SEQ ID NO:4, respectively. DNAs encoding these polypeptides are shown in SEQ ID NOS:1 and 3, respectively.

The term "zvegf3 protein" is used herein to denote proteins comprising the growth factor domain of a zvegf3 polypeptide (e.g., residues 235-345 of human zvegf3 (SEQ ID NO:2) or mouse zvegf3 (SEQ ID NO:4)), wherein the protein is mitogenic for cells expressing cell-surface PDGF α-receptor subunit. Zvegf3 has been found to bind to the αα and αβ isoforms of PDGF receptor. Zvegf3 is a homodimeric protein that is naturally produced as a precursor that is proteolytically activated to release the mature protein, a dimer of the growth factor domain. As used herein, "zvegf3 protein" includes precursors that are activatable *in vivo.* Using methods known in the art, zvegf3 proteins can be prepared in a variety of forms, including glycosylated or non-glycosylated, pegylated or non-pegylated, with or without an initial methionine residue, and as fusion proteins as disclosed in more detail below.

The zvegf3 polypeptide chain comprises a growth factor domain and a CUB domain. The growth factor domain is characterized by an arrangement of cysteine residues and beta strands that is characteristic of the "cystine knot" structure of the PDGF family. The CUB domain shows sequence homology to CUB domains in the neuropilins (Takagi et al., Neuron 7:295-307, 1991; Soker et al., Cell 92:735-745, 1998), human bone morphogenetic protein-1 (Wozney et al., Science 242:1528-1534, 1988), porcine seminal plasma protein and bovine acidic seminal fluid protein (Romero et al., Nat. Struct. Biol. 4:783-788, 1997), and *X*. *laevis* tolloid-like protein (Lin et al., Dev. Growth Differ. 39:43-51, 1997).

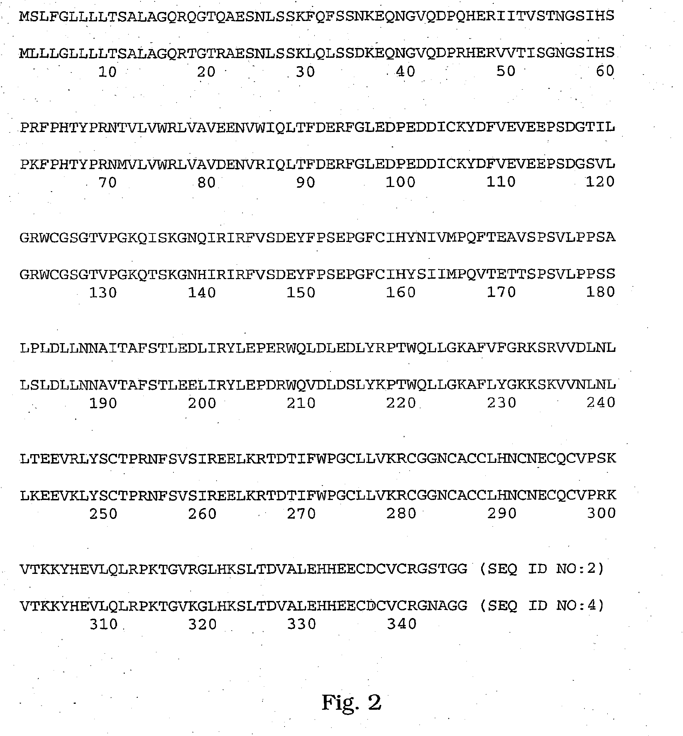

An alignment of mouse and human zvegf3 polypeptide sequences is shown in Fig. 2. Analysis of the amino acid sequence shown in SEQ ID NO:2 indicates that residues 1 to 14 form a secretory peptide. The CUB domain extends from residue 46 to residue 163. A propeptide-like sequence extends from residue 164 to residue 234, and includes two potential cleavage sites at its carboxyl terminus, a dibasic site at residues 231-232 and a target site for furin or a furin-like protease at residues 231-234. The growth factor domain extends from residue 235 to residue 345. Those skilled in the art will recognize that domain boundaries are somewhat imprecise and can be expected to vary by up to ± 5 residues from the specified positions. Potential proteolytic cleavage sites occur at residues 232 and 234. Processing of recombinant zvegf3 produced in BHK cells has been found to occur between residues 225 and 226. Signal peptide cleavage is predicted to occur after residue 14 (± 3 residues). This analysis suggests that the zvegf3 polypeptide chain may be cleaved to produce a plurality of monomeric species as shown in Table 1. Cleavage after Arg-234 is expected to result in subsequent removal of residues 231-234, with possible conversion of Gly-230 to an amide. Cleavage after Lys-232 is expected to result in subsequent removal of residue 231, again with possible conversion of Gly-230 to an amide. In addition, it may be advantageous to include up to seven residues of the interdomain region at the carboxyl terminus of the CUB domain. The interdomain region can be truncated at its amino terminus by a like amount. See

### Table 1. Corresponding domains in mouse and other non-human zvegf3s can be determined by those of ordinary skill in the art from sequence alignments.

**Table 1**

| Monomer | Residues (SEQ ID NO:2) |
|---|---|
| Cub domain | 15-163 |
| | 46-163 |
| | 15-170 |
| | 46-170 |
| | |
| CUB domain + interdomain region | 15-234 |
| | 46-234 |
| | 15-229 amide |
| | 15-230 |
| | |
| Cub domain + interdomain region + growth factor domain | 15-345 |
| | 46-345 |
| | |
| Growth factor domain | 235-345 |
| | 226-345 |
| | |
| Growth factor domain + interdomain region | 164-345 |
| | 171- 345 |

Zvegf3 can thus be prepared in a variety of multimeric forms comprising a zvegf3 polypeptide as disclosed above. These zvegf3 polypeptides include zvegf3₁₅₋₃₄₅ , zvegf3₄₆₋₃₄₅, zvegf3₂₂₆₋₃₄₅, and zvegf₃₂₃₅₋₃₄₅. Variants and derivatives of these polypeptides can also be prepared as disclosed herein. Intermediate forms can also be produced. For example, a growth factor domain polypeptide may have, as an amino-terminal residue, any of residues 226-240 of SEQ ID NO:2, inclusive.

Zvegf3 proteins can be prepared as fusion proteins comprising amino- or carboxyl-terminal extensions, such as an amino-terminal methionine residue, an affinity tag, or a targetting polypeptide. For example, a zvegf3 protein can be prepared as a fusion with an affinity tag to facilitate purification. In principal, any peptide or protein for which an antibody or other specific binding agent is available can be used as an affinity tag. Affinity tags include, for example, a poly-histidine tract, protein A (Nilsson et al., EMBO J. 4:1075, 1985; Nilsson et al., Methods Enzymol. 198:3, 1991), glutathione S transferase (Smith and Johnson, Gene 67:31, 1988), Glu-Glu affinity tag (Grussenmeyer et al., Proc. Natl. Acad. Sci. USA 82:7952-4, 1985), substance P, FLAG peptide (Hopp et al., Biotechnology 6:1204-1210, 1988), streptavidin binding peptide, maltose binding protein (Guan et al., Gene 67:21-30, 1987), cellulose binding protein, thioredoxin, ubiquitin, T7 polymerase, or other antigenic epitope or binding domain. Fusion of zvegf3 to, for example, maltose binding protein or glutathione S transferase can be used to improve yield in bacterial expression systems. In these instances the non-zvegf3 portion of the fusion protein ordinarily will be removed prior to use. Separation of the zvegf3 and non-zvegf3 portions of the fusion protein is facilitated by providing a specific cleavage site between the two portions. Such methods are well known in the art. Zvegf3 can also be fused to a targetting peptide, such as an antibody (including polyclonal antibodies, monoclonal antibodies, antigen-binding fragments thereof such as F(ab')₂ and Fab fragments, single chain antibodies, and the like) or other peptidic moiety that binds to a target tissue.

Variations can be made in the zvegf3 amino acid sequences shown in SEQ ID NO:2 and SEQ ID NO:4 to provide mitogenically inactive, receptor-binding polypeptides that act as zvegf3 antagonists. As used herein, the term "mitogenically inactive" means that the protein does not show statistically significant activity in a standard mitogenesis assay as compared to a wild-type zvegf3 control. Such variations include amino acid substitutions, deletions, and insertions. While not wishing to be bound by theory, it is believed that residues Arg260-Trp271 of human zvegf3 (SEQ ID NO:2) form a loop that defines the ability of the protein to bind to PDGF receptors. It is thus predicted that binding to PDGF receptors can be blocked or enhanced by mutations in this region. In addition, residues Leu311-His321 of SEQ ID NO:2 are predicted to form a loop (loop3) that may be mutated to block receptor binding. Peptides that mimic this region of the molecule may act as antagonists. Li et al. (Cytokine & Growth Factor Rev. 14:91-98, 2003) disclose that partially processed forms of zvegf3 in which only one of the two CUB domains is removed from the homodimeric molecule (termed "hemi-dimers") may be able to bind PDGF receptors but block receptor dimerization.

The effects of amino acid sequence changes can be predicted by computer modeling (using, e.g., the Insight II® viewer and homology modeling tools; MSI, San Diego, CA) or determined by analysis of crystal structure (see, e.g., Lapthorn et al., Nature 369:455, 1994), and can be assessed using art-recognized mutagenesis procedures in combination with activity assays. Representative mutagenesis procedures include, for example, site-directed mutagenesis and alanine-scanning mutagenesis (Cunningham and Wells, Science 244, 1081-1085, 1989; Bass et al., Proc. Natl. Acad. Sci. USA 88:4498-4502, 1991). Multiple amino acid substitutions can be made and tested using known methods, such as those disclosed by Reidhaar-Olson and Sauer (Science 241:53-57, 1988) or Bowie and Sauer (Proc. Natl. Acad. Sci. USA 86:2152-2156, 1989). Other methods that can be used include phage display (e.g., Lowman et al., Biochem. 30:10832-10837, 1991; Ladner et al.,U.S. Patent No. 5,223,409; Huse, WIPO Publication WO 92/06204), region-directed mutagenesis (Derbyshire et al., Gene 46:145, 1986; Ner et al., DNA 7:127, 1988), and DNA shuffling as disclosed by Stemmer (Nature 370:389-391, 1994) and Stemmer (Proc. Natl. Acad. Sci. USA 91:10747-10751, 1994). The resultant mutant molecules are tested for mitogenic activity or other properties (e.g., receptor binding) to identify amino acid residues that are critical to these functions. Mutagenesis can be combined with high volume or high-throughput screening methods to detect biological activity of zvegf3 variant polypeptides, in particular biological activity in modulating cell proliferation. For example, mitogenesis assays that measure dye incorporation or ³H-thymidine incorporation can be carried out on large numbers of samples. Competition assays can be employed to confirm antagonist activity.

Zvegf3 proteins, including full-length polypeptides, fragments, and fusions proteins, as well as antagonist variants, can be produced in genetically engineered host cells according to conventional techniques. Suitable host cells are those cell types that can be transformed or transfected with exogenous DNA and grown in culture, and include bacteria, fungal cells, and cultured higher eukaryotic cells (including cultured cells of multicellular organisms). Techniques for manipulating cloned DNA molecules and introducing exogenous DNA into a variety of host cells are disclosed by Sambrook et al., Molecular Cloning: A Laboratory Manual, 2nd ed., Cold Spring Harbor Laboratory Press, Cold Spring Harbor, NY, 1989, and Ausubel et al., eds., Current Protocols in Molecular Biology, Green and Wiley and Sons, NY, 1993. See, WHO 00/34474. In general, a DNA sequence encoding a zvegf3 polypeptide is operably linked to other genetic elements required for its expression, generally including a transcription promoter and terminator, within an expression vector. The vector will also commonly contain one or more selectable markers and one or more origins of replication, although those skilled in the art will recognize that within certain systems selectable markers may be provided on separate vectors, and replication of the exogenous DNA may be provided by integration into the host cell genome. Selection of promoters, terminators, selectable markers, vectors and other elements is a matter of routine design within the level of ordinary skill in the art. Many such elements are described in the literature and are available through commercial suppliers. See, WO 00/34474.

Zvegf3 proteins and variants can also comprise non-naturally occurring amino acid residues. Non-naturally occurring amino acids include, without limitation, trans-3-methylproline, 2,4-methanoproline, cis-4-hydroxyproline, *trans*-4-hydroxyproline, N-methylglycine, allo-threonine, methylthreonine, hydroxyethylcysteine, hydroxyethylhomocysteine, nitroglutamine, homoglutamine, pipecolic acid, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into proteins. For example, an *in vitro* system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell-free system comprising an *E. coli* S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-809, 1993; and Chung et al., Proc. Natl. Acad. Sci. USA 90:10145-10149, 1993). In a second method, translation is carried out in Xenopus oocytes by microinjection of mutated mRNA and chemically aminoacylated suppressor tRNAs (Turcatti et al., J. Biol. Chem. 271:19991-19998, 1996). Within a third method, *E. coli* cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the protein in place of its natural counterpart. See, Koide et al., Biochem. 33:7470-7476, 1994. Naturally occurring amino acid residues can be converted to non-naturally occurring species by *in vitro* chemical modification. Chemical modification can be combined with site-directed mutagenesis to further expand the range of substitutions (Wynn and Richards, Protein Sci. 2:395-403, 1993).

Zvegf3 polypeptides, fragments, or variants thereof can also be prepared through chemical synthesis according to methods known in the art, including exclusive solid phase synthesis, partial solid phase methods, fragment condensation or classical solution synthesis. See, for example, Merrifield, J. Am. Chem. Soc. 85:2149, 1963; Stewart et al., Solid Phase Peptide Synthesis (2nd edition), Pierce Chemical Co., Rockford, IL, 1984; Bayer and Rapp, Chem. Pept. Prot. 3:3, 1986; and Atherton et al., Solid Phase Peptide Synthesis: A Practical Approach, IRL Press, Oxford, 1989.

Zvegf3 proteins and variants thereof are purified by conventional protein purification methods, typically by a combination of chromatographic techniques. See, in general, Affinity Chromatography: Principles & Methods, Pharmacia LKB Biotechnology, Uppsala, Sweden, 1988; and Scopes, Protein Purification: Principles and Practice, Springer-Verlag, New York, 1994. Proteins comprising a polyhistidine affinity tag (typically about 6 histidine residues) are purified by affinity chromatography on a nickel chelate resin. See, for example, Houchuli et al., Bio/Technol. 6: 1321-1325, 1988. Furthermore, the growth factor domain itself binds to nickel resin at pH 7.0-8.0 and 25 mM Na phosphate, 0.25 M NaCl. Bound protein can be eluted with a descending pH gradient down to pH 5.0 or an imidazole gradient. Recombinant zvegf3 growth factor domain protein can be purified using a combination of chromatography on a strong cation exchanger followed by a tandem column array comprising a strong anion exchanger followed by an immobilized metal affinity column in series. It has also been found that zvegf3 binds to various dye matrices (e.g., BLUE1, BLUE 2, ORANGE 1, ORANGE 3, and RED3 from Lexton Scientific, Signal Hill, CA) in PBS at pH 6-8, from which the bound protein can be eluted in 1-2 M NaCl in 20 mM boric acid buffer at pH 8.8. Protein eluted from RED3 may be passed over RED2 (Lexton Scientific) to remove remaining contaminants. Proteins comprising a glu-glu tag can be purified by immunoaffinity chromatography according to conventional procedures. See, for example, Grussenmeyer et al., *ibid.* Maltose binding protein fusions are purified on an amylose column according to methods known in the art.

As disclosed in more detail below, overexpression of zvegf3 in the livers of transgenic mice led to marked stellate cell activation and proliferation. At 8 weeks of age there was an accumulation of perisinusiodal extracellular matrix (ECM) that progressed to a perivenular ECM deposition at 22 weeks and possible early stages of cirrhosis characterized by fibrotic banding and hepatic nodule formation at 33 weeks of age. Older animals were found to have hemagioendothelioma, nodular hepatocellular hyperplasia, and hepatocellular adenoma. These changes are consistent with the development of hepatocellular carcinoma as a result of long-term overexpression of zvegf3.

Also as disclosed in more detail below, stimulation of rat hepatic stellate cells with zvegf3 growth factor domain protein resulted in an approximately 5-fold increase in the production of TGF-β compared to control cells. TGF-β is believed to be an important modulator of hepatic fibrosis that can induce the expression of other pro-fibrotic genes. Hence, the effects observed in animals overexpressing zvegf3 may be due to both direct and indirect effects of zvegf3.

Zvegf3 antagonists include, without limitation, anti-zvegf3 antibodies (including neutralizing antibodies), inhibitory polynucleotides (including antisense polynucleotides, ribozymes, and external guide sequences), and other peptidic and non-peptidic agents, including small molecule inhibitors and mitogenically inactive receptor-binding zvegf3 polypeptides.

Antibodies used as zvegf3 antagonists include antibodies that specifically bind to a zvegf3 protein and, by so binding, reduce or prevent the binding of zvegf3 protein to the receptor and, consequently, reduce or block the receptor-mediated activity of zvegf3. As used herein, the term "antibodies" includes polyclonal antibodies, affinity-purified polyclonal antibodies, monoclonal antibodies, and antigen-binding fragments, such as F(ab')₂ and Fab proteolytic fragments. Genetically engineered intact antibodies or fragments, such as chimeric antibodies, Fv fragments, single chain antibodies and the like, as well as synthetic antigen-binding peptides and polypeptides, are also included. Non-human antibodies may be humanized by grafting non-human CDRs onto human framework and constant regions, or by incorporating the entire non-human variable domains (optionally "cloaking" them with a human-like surface by replacement of exposed residues, wherein the result is a "veneered" antibody). In some instance; humanized antibodies may retain non-human residues within the human variable region framework domains to enhance proper binding characteristics. Through humanizing antibodies, biological half-life may be increased, and the potential for adverse immune reactions upon administration to humans is reduced. Monoclonal antibodies can also be produced in mice that have been genetically altered to produce antibodies that have a human structure. IgG class antibodies are generally preferred for use within the present invention.

Methods for preparing and isolating polyclonal and monoclonal antibodies are well known in the art. See, for example, Cooligan et al. (eds.), Current Protocols in Immunology, National Institutes of Health, John Wiley and Sons, Inc., 1995; Sambrook et al., Molecular Cloning: A Laboratory Manual, second edition, Cold Spring Harbor, NY, 1989; and Hurrell (ed.), Monoclonal Hybridoma Antibodies: Techniques and Applications, CRC Press, Inc., Boca Raton, FL, 1982. As would be evident to one of ordinary skill in the art, polyclonal antibodies can be generated by inoculating a variety of warm-blooded animals such as horses, cows, goats, sheep, dogs, chickens, rabbits, mice, and rats with a zvegf3 polypeptide or a fragment thereof.

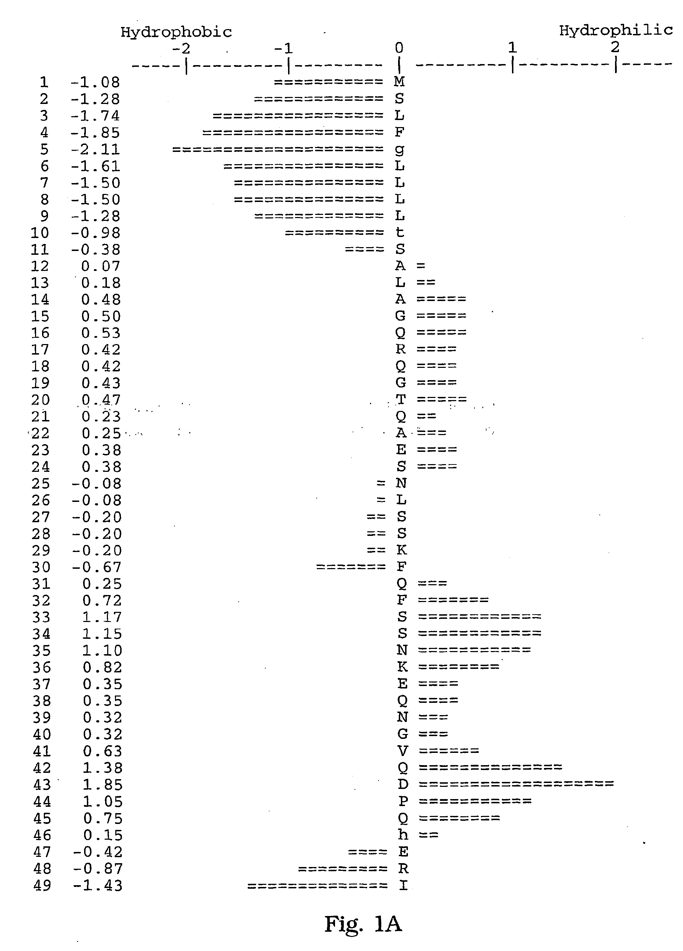

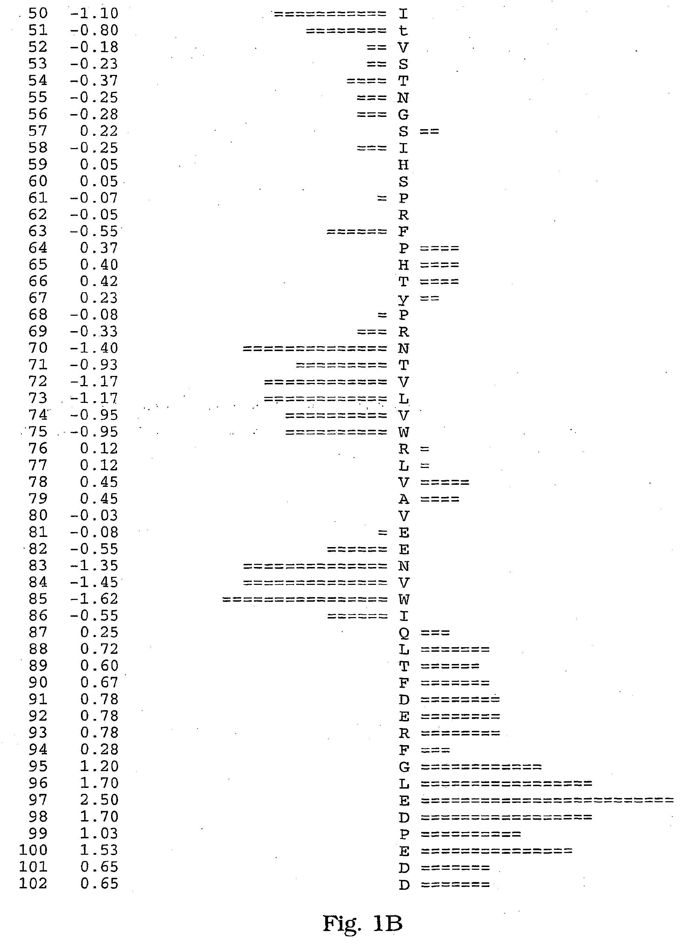

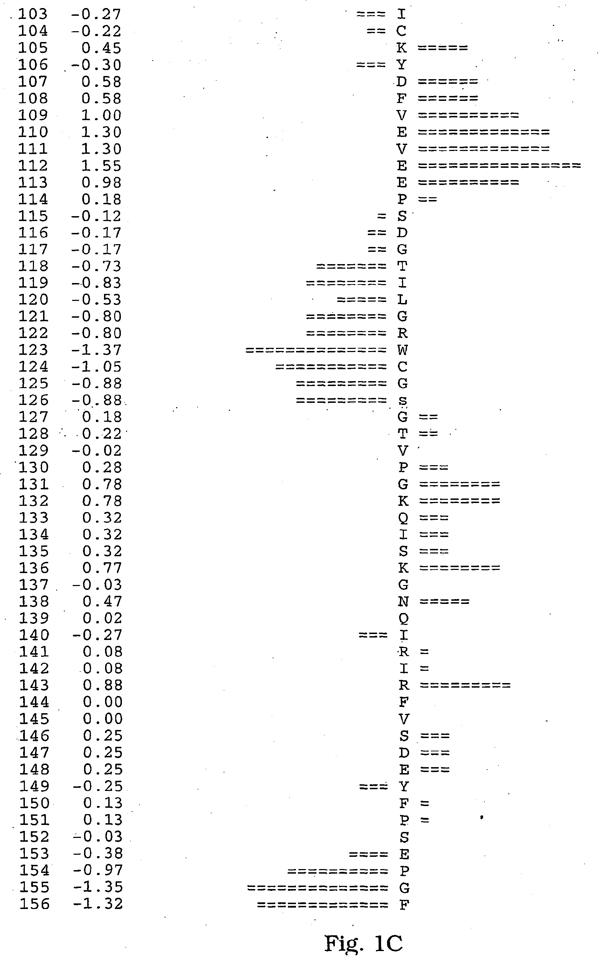

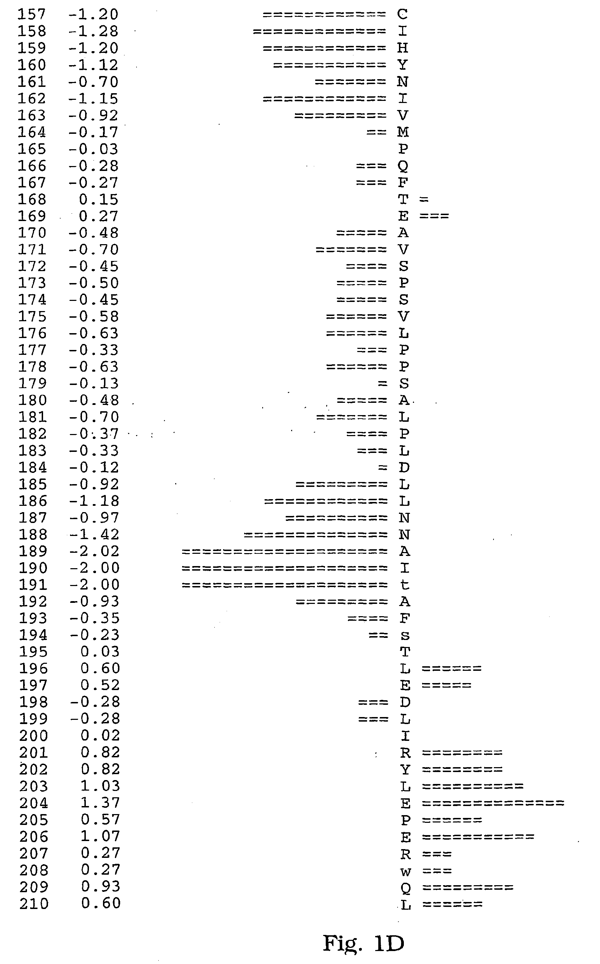

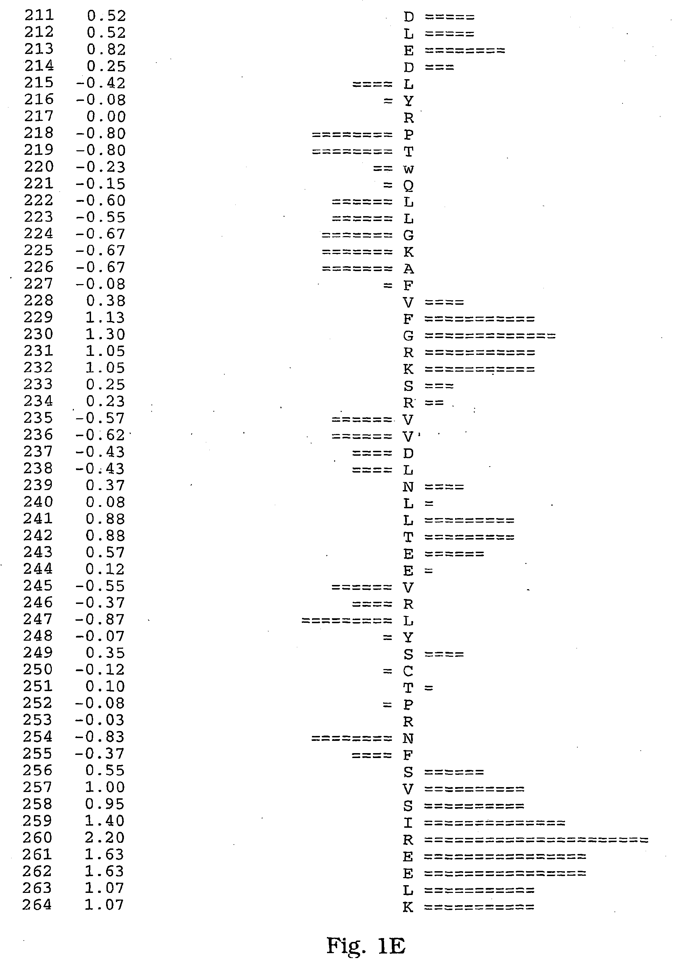

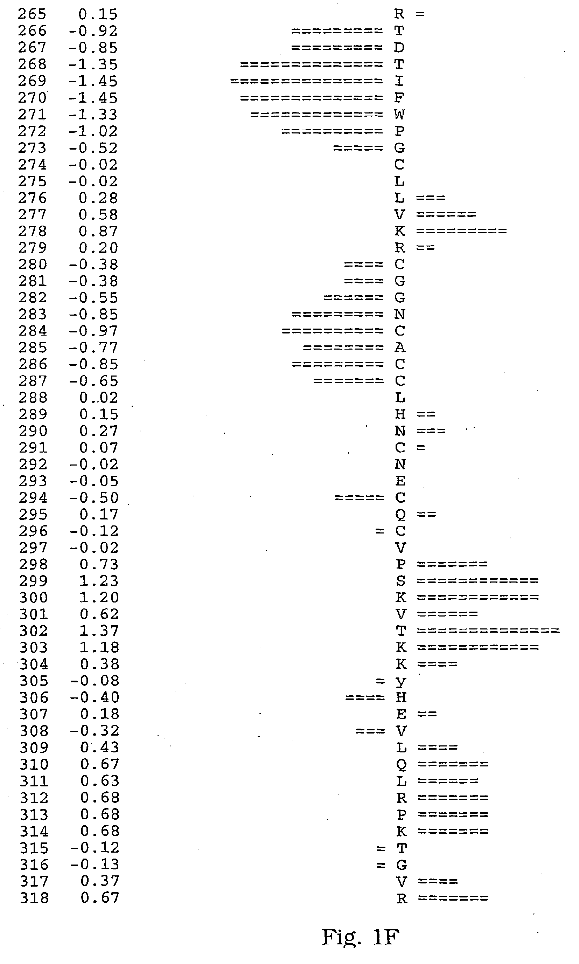

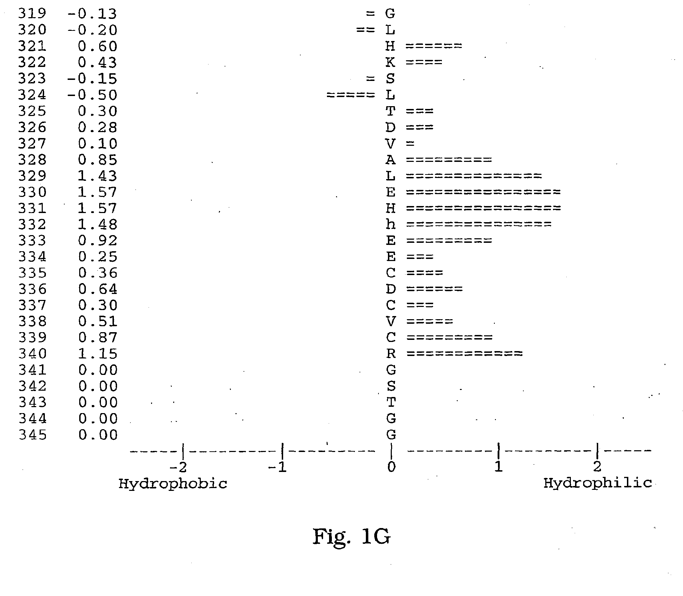

Immunogenic polypeptides will comprise an epitope-bearing portion of a zvegf3 polypeptide (e.g., as shown in SEQ ID NO:2) or receptor. An "epitope" is a region of a protein to which an antibody can bind. See, for example, Geysen et al., Proc. Natl. Acad. Sci. USA 81:3998-4002, 1984. Epitopes can be linear or conformational, the latter being composed of discontinuous regions of the protein that form an epitope upon folding of the protein. Linear epitopes are generally at least 6 amino acid residues in length. Relatively short synthetic peptides that mimic part of a protein sequence are routinely capable of eliciting an antiserum that reacts with the partially mimicked protein. See, Sutcliffe et al., Science 219:660-666, 1983. Immunogenic, epitope-bearing polypeptides contain a sequence of at least six, often at least nine, more often from 15 to about 30 contiguous amino acid residues of a zvegf3 polypeptide or receptor. Polypeptides comprising a larger portion of a zvegf3. protein or receptor, i.e. from 30 to 50 residues up to the entire sequence, are included. It is preferred that the amino acid sequence of the epitope-bearing polypeptide is selected to provide substantial solubility in aqueous solvents, that is the sequence includes relatively hydrophilic residues, and hydrophobic residues are substantially avoided. See Figs. 1A - 1G. Such regions include residues 43-48, 96-101, 97-102, 260-265, and 330-335 of SEQ ID NO:2. As noted above, it is generally preferred to use somewhat longer peptides as immunogens, such as a peptide comprising residues 80-104, 195-225, 299-314, and 299-326 of SEQ ID NO:2. The latter peptide can be prepared with an additional N-terminal Cys residue to facilitate coupling.

The immunogenicity of a polypeptide immunogen may be increased through the use of an adjuvant, such as alums (aluminum hydroxide) or Freund's complete or incomplete adjuvant. Polypeptides useful for immunization also include fusion polypeptides, such as fusions of a zvegf3 polypeptide or a portion thereof with an immunoglobulin polypeptide or with maltose binding protein. If the polypeptide portion is "hapten-like", such portion may be advantageously joined or linked to a macromolecular carrier (such as keyhole limpet hemocyanin (KLH), bovine serum albumin (BSA), or tetanus toxoid) for immunization.

Alternative techniques for generating or selecting antibodies include *in vitro* exposure of lymphocytes to a polypeptide immunogen, and selection of antibody display libraries in phage or similar vectors (for instance, through use of immobilized or labeled polypeptide). Techniques for creating and screening such random peptide display libraries are known in the art (e.g., Ladner et al., US Patent No. 5,223,409; Ladner et al., US Patent No. 4,946,778; Ladner et al., US Patent No. 5,403,484 and Ladner et al., US Patent No. 5,571,698), and random peptide display libraries and kits for screening such libraries are available commercially, for instance from Clontech Laboratories (Palo Alto, CA), Invitrogen Inc. (San Diego, CA), New England Biolabs, Inc. (Beverly, MA), and Pharmacia LKB Biotechnology Inc. (Piscataway, NJ). Random peptide display libraries can be screened using the zvegf3 sequences disclosed herein to identify proteins which bind to zvegf3.

Antibodies are determined to be specifically binding if they bind to their intended target (e.g., zvegf3 protein or receptor) with an affinity at least 10-fold greater than the binding affinity to control (e.g., non-zvegf3 or non-receptor) polypeptide or protein. In this regard, a "non-zvegf3 polypeptide" includes the related molecules VEGF, VEGF-B, VEGF-C, VEGF-D, P1GF, PDGF-A, and PDGF-B, but excludes zvegf3 polypeptides from non-human species. Due to the high level of amino acid sequence identity expected between zvegf3 orthologs, antibodies specific for human zvegf3 may also bind to zvegf3 from other species. The binding affinity of an antibody can be readily determined by one of ordinary skill in the art, for example, by Scatchard analysis (Scatchard, G., Ann. NY Acad. Sci. 51: 660-672, 1949). Methods for screening and isolating specific antibodies are well known in the art. See, for example, Paul (ed.), Fundamental Immunology, Raven Press, 1993; Getzoff et al., Adv. in Immunol. 43:1-98, 1988; Goding (ed.), Monoclonal Antibodies: Principles and Practice, Academic Press Ltd., 1996; Benjamin et al., Ann. Rev. Immunol. 2:67-101, 1984.

Binding affinity can also be determined using a commercially available biosensor instrument (BIACORE, Pharmacia Biosensor, Piscataway, NJ), wherein protein is immobilized onto the surface of a receptor chip. See, Karlsson, J. Immunol. Methods 145:229-240, 1991 and Cunningham and Wells, J. Mol. Biol. 234:554-563, 1993. This system allows the determination of on and off-rates, from which binding affinity can be calculated, and assessment of stoichiometry of binding.

Antibodies are considered to be "isolated" when they are prepared essentially free of other antibodies of different specificity. Use of isolated antibodies facilitates targeting of specific epitopes or portions of zvegf3, such as the growth factor domain.

A variety of assays known to those skilled in the art can be utilized to detect antibodies that specifically bind to zvegf3 proteins or receptors. Exemplary assays are described in detail in Antibodies: A Laboratory Manual, Harlow and Lane (Eds.), Cold Spring Harbor Laboratory Press, 1988. Representative examples of such assays include: concurrent immunoelectrophoresis, radioimmunoassay, radioimmunoprecipitation, enzyme-linked immunosorbent assay (ELISA), dot blot or Western blot assays, inhibition or competition assays, and sandwich assays.

For therapeutic applications it is generally preferred to use neutralizing antibodies. As used herein, the term "neutralizing antibody" denotes an antibody that inhibits at least 50% of the biological activity of the cognate antigen when the antibody is added at a 1000-fold molar access. Those of skill in the art will recognize that greater neutralizing activity is sometimes desirable, and antibodies that provide 50% inhibition at a 100-fold or 10-fold molar access may be advantageously employed.

Zvegf3 antagonists further include antisense polynucleotides, which can be used to inhibit zvegf3 gene transcription and thereby inhibit cell activation and/or proliferation *in vivo*. Polynucleotides that are complementary to a segment of a zvegf3-encoding polynucleotide (e.g., a polynucleotide as set forth in SEQ ID NO:1) are designed to bind to zvegf3-encoding mRNA and to inhibit translation of such mRNA. Antisense polynucleotides can be targetted to specific tissues using a gene therapy approach with specific vectors and/or promoters, such as viral delivery systems as disclosed in more detail below.

Ribozymes can also be used as zvegf3 antagonists. Ribozymes are RNA molecules that contain a catalytic center and a target RNA binding portion. The term includes RNA enzymes, self-splicing RNAs, self-cleaving RNAs, and nucleic acid molecules that perform these catalytic functions. A ribozyme selectively binds to a target RNA molecule through complementary base pairing, bringing the catalytic center into close proximity with the target sequence. The ribozyme then cleaves the target RNA and is released, after which it is able to bind and cleave additional molecules. A nucleic acid molecule that encodes a ribozyme is termed a "ribozyme gene." Ribozymes can be designed to express endonuclease activity that is directed to a certain target sequence in a mRNA molecule (see, for example, Draper and Macejak, U.S. Patent No. 5,496,698; McSwiggen, U.S. Patent No. 5,525,468; Chowrira and McSwiggen, U.S. Patent No. 5,631,359; and Robertson and Goldberg, U.S. Patent No. 5,225,337). An expression vector can be constructed in which a regulatory element is operably linked to a nucleotide sequence that encodes a ribozyme.

In another approach, expression vectors can be constructed in which a regulatory element directs the production of RNA transcripts capable of promoting RNase P-mediated cleavage of mRNA molecules that encode a zvegf3 polypeptide. According to this approach, an external guide sequence can be constructed for directing the endogenous ribozyme, RNase P, to a particular species of intracellular mRNA, which is subsequently cleaved by the cellular ribozyme (see, for example, Altman et al., U.S. Patent No. 5,168,053; Yuan et al., Science 263:1269, 1994; Pace et al., WIPO Publication No. WO 96/18733; George et al., WIPO Publication No. WO 96/21731; and Werner et al., WIPO Publication No. WO 97/33991). An external guide sequence generally comprises a ten- to fifteen-nucleotide sequence complementary to zvegf3 mRNA, and a 3'-NCCA nucleotide sequence, wherein N is preferably a purine. The external guide sequence transcripts bind to the targeted mRNA species by the formation of base pairs between the mRNA and the complementary external guide sequences, thus promoting cleavage of mRNA by RNase P at the nucleotide located at the 5'-slide of the base-paired region.

The growth factor domain of zvegf3 has been found to be the active (PDGF receptor-binding) species of the molecule. Proteolytic processing to remove the N-terminal portion of the molecule is required for activation. Thus, inhibitors of this proteolytic activation can also be used as zvegf3 antagonists.

For pharmaceutical use, zvegf3 antagonists are formulated for parenteral, particularly intravenous or intraperitoneal, delivery according to conventional methods. In general, pharmaceutical formulations will include a zvegf3 antagonist in combination with a pharmaceutically acceptable vehicle, such as saline, buffered saline, 5% dextrose in water, 5% human serum albumin, or the like. Formulations may further include one or more excipients, preservatives, solubilizers, buffering agents, albumin to prevent protein loss on vial surfaces, etc. Liposomes may be used as carriers according to known procedures. Methods of formulation are well known in the art and are disclosed, for example, in Remington: The Science and Practice of Pharmacy, Gennaro, ed., Mack Publishing Co., Easton, PA, 19th ed., 1995. A zvegf3 antagonist will normally be formulated and packaged in unit dose form. Antibodies will typically be formulated at concentrations of about 1 mg/ml to about 10 mg/ml. A "therapeutically effective .. amount" of a thereapeutic agent or composition is that amount that produces a statistically significant effect, such as a statistically significant increase in survival rate over a given time period, increase in survival time, reduction in tumor mass, reduction in tumor metastasis, reduction in disease progression, reduction in time to progression, and the like. The exact dose will be determined by the clinician according to accepted standards, taking into account the nature and severity of the condition to be treated, patient traits, etc. Determination of dose is within the level of ordinary skill in the art. The therapeutic formulations will generally be administered over the period required to achieve a beneficial effect, commonly up to several months. Dosing is daily or intermittently over the period of treatment. Intravenous administration ordinarily will be by infusion over a typical period of one to several hours. Sustained release formulations can also be employed. A large loading dose may be administered initially, followed by smaller, periodic, maintenance doses.

Other mitogenic factors, including hepatocyte growth factor (HGF), vascular endothelial growth factor (VEGF), and insulin-like growth factor (IGF), have been implicated in the initiation or progression of hepatocellular carcinoma. It may therefore be advantageous to combine a zvegf3 inhibitor with one or more inhibitors of these other factors.

A zvegf3 antagonist can be administered in combination with known therapies for hepatocellular carcinoma, including surgery, chemotherapy, radiofrequency ablation, laser or microwave therapy, percutaneous ethanol injection, cryosurgery, immunotherapy, or combinations thereof. For example, a zvegf3 antagonist can be administered in combination with conventional chemotherapeutic agents such as cisplatin (Epstein et al., Cancer 67:896-900, 1991), doxorubicin hydrochloride (Choi et al, Radiology 182:709-713, 1992), 5-fluorodeoxyuridine (Ensminger et al., Cancer Treat Rep. 65:393-400, 1981), dichloromethotrexate (Ensminger et al., ibid.), or bischlorethylnitrosourea (Dakhil et al., Cancer 50:631-635, 1982). Regional selectivity of chemotherapy may be enhanced and systemic toxicity reduced through chemoembolization (e.g., Choi et al., ibid.; Dakhil et al., ibid.) or hepatic arterial infusion (e.g., Ensminger et al., ibid.). Administration of multiple therapeutic agents can be simultaneous or sequential. Chemoembolization, cryosurgery, percutaneous ethanol injection, and radiofrequency ablation are of particular interest in the treatment of smaller (< 5 cm), localized tumors that are unresectable due to location or the presence of other medical conditions.

Within certain embodiments of the invention administration of zvegf3 antagonists alone or in combination with other therapy will result in a tumor response. While each protocol may define tumor response assessments differently, exemplary guideline can be found in Clinical Research Associates Manual; Southwest Oncology Group, CRAB, Seattle, WA; October 6, 1998, updated August 1999 ("CRA Manual"). According to the CRA Manual (see, Chapter 7, "Response Accessment"), tumor response means a reduction or elimination of all measurable lesions or metastases. Disease is generally considered measurable if it comprises bidimensionally measurable lesions with clearly defined margins by medical photograph or X-ray, computerized axial tomography (CT), magnetic resonance imaging (MRI), or palpation. Evaluable disease means the disease comprises unidimensionally measurable lesions, masses with margins not clearly defined, lesion with both diameters less than 0.5 cm, lesions on scan with either diameter smaller than the distance between cuts, palpable lesions with diameter less than 2 cm, or bone disease. Non-evaluable disease includes pleural effusions, ascites, and disease documented by indirect evidence. Previously radiated lesions which have not progressed are also generally considered non-evaluable.

Criteria for objective status are required for protocols to assess solid tumor response. Representative criteria includes the following: (1) Complete Response (CR), defined as complete disappearance of all measurable and evaluable disease. No new lesions. No disease related symptoms. No evidence of non-evaluable disease; (2) Partial Response (PR), defined as greater than or equal to 50% decrease from baseline in the sum of products of perpendicular diameters of all measurable lesions. No progression of evaluable disease. No new lesions. Applies to patients with at least one measurable lesion; (3) Progression, defined as 50% or an increase of 10 cm² in the sum of products of measurable lesions over the smallest sum observed using same techniques as baseline, or clear worsening of any evaluable disease, or reappearance of any lesion which had disappeared, or appearance of any new lesion, or failure to return for evaluation due to death or deteriorating condition (unless unrelated to this cancer); (4) Stable or No Response, defined as not qualifying for CR, PR, or Progression. (See, Clinical Research Associates Manual, supra.).

Antibodies are preferably administered parenterally, generally by intravenous infusion (including hepatic arterial infusion) over the course of treatment. Administration may also be intraperitoneal. Antibodies are generally administered in the range of about 0.1 to about 20 mg/kg of patient weight, commonly about 0.5 to about 10 mg/kg, and often about 1 to about 5 mg/kg. In this regard, it is preferred to use antibodies having a circulating half-life of at least 12 hours, preferably at least 4 days, more preferably up to 14-21 days. Chimeric and humanized antibodies are expected to have circulatory half-lives of up to four and up to 14-21 days, respectively. In many cases it will be preferable to administer a large loading dose followed by periodic (e.g., weekly) maintenance doses over the treatment period. Antibodies can also be delivered by slow-release delivery systems, pumps, and other known delivery systems for continuous infusion. Dosing regimens may be varied to provide the desired.circulating levels of a particular antibody based on its pharmacokinetics. Thus, doses will be calculated so that the desired circulating level of therapeutic agent is maintained.

The actual dose and treatment regimen will be' determined by the physician, taking into account the nature of the cancer (primary or metastatic), number and size of tumors, other therapies, and patient characteristics. In view of the life-threatening nature of hepatocellular carcinoma, large doses with significant side effects may be employed.

Those skilled in the art will recognize that the same principles will guide the use of other zvegf3 antagonists. The dosing regimen for a given antagonist will be determined by a number of factors including potency, pharmacokinetics, and the physicochemical nature of the antagonist. For example, non-peptidic zvegf3 antagonists may be administered enterally.

Therapeutic polynucleotides, such as antisense polynucleotides, can be delivered to patients or test animals by way of viral delivery systems. Exemplary viruses for this purpose include adenovirus, herpesvirus, retroviruses, vaccinia virus, and adeno-associated virus (AAV). Adenovirus, a double-stranded DNA virus, is currently the best studied gene transfer vector for delivery of heterologous nucleic acids. For review, see Becker et al., Meth. Cell Biol. 43:161-189, 1994; and Douglas and Curiel, Science & Medicine 4:44-53, 1997. The adenovirus system offers several advantages. Adenovirus can (i) accommodate relatively large DNA inserts; (ii) be grown to high-titer; (iii) infect a broad range of mammalian cell types; and (iv) be used with many different promoters including ubiquitous, tissue specific, and regulatable promoters. Because adenoviruses are stable in the bloodstream, they can be administered by intravenous injection.

By deleting portions of the adenovirus genome, larger inserts (up to 7 kb) of heterologous DNA can be accommodated. These inserts can be incorporated into the viral DNA by direct ligation or by homologous recombination with a co-transfected plasmid. When intravenously administered to intact animals, adenovirus primarily targets the liver. If the adenoviral delivery system has an E1 gene deletion, the virus cannot replicate in the host cells. However, the host's tissue (e.g., liver) will express and process (and, if a signal sequence is present, secrete) the heterologous protein.

An alternative method of gene delivery comprises removing cells from the body and introducing a vector into the cells as a naked DNA plasmid. The transformed cells are then re-implanted in the body. Naked DNA vectors are introduced into host cells by methods known in the art, including transfection, electroporation; microinjection, transduction, cell fusion, DEAE dextran, calcium phosphate precipitation, use of a gene gun, or use off a DNA vector transporter. See, Wu et al., J. Biol. Chem: 263:14621-14624, 1988; Wu et al., J. Biol. Chem. 267:963-967, 1992; and Johnston and Tang, Meth. Cell Biol. 43:353-365, 1994.

Activity of zvegf3 antagonists can be measured *in vitro* using assays (including cell-based assays) designed to measure zvegf3 activity. Antagonists will reduce the effects of zvegf3 within the assay. Ligand-receptor binding can be assayed by a variety of methods well known in the art, including receptor competition assays (Bowen-Pope and Ross, Methods Enzyme. 109:69-100, 1985) and through the use of soluble receptors, including receptors produced as IgG fusion proteins (U.S. Patent No. 5,750,375). Receptor binding assays can be performed on cell lines that contain known cell-surface receptors for evaluation. The receptors can be naturally present in the cell, or can be recombinant receptors expressed by genetically engineered cells. Mitogenic activity can be measured using known assays, including ³H-thymidine incorporation assays (as disclosed by, e.g., Raines and Ross, Methods Enzymol. 109:749-773, 1985 and Wahl et al., Mol. Cell Biol. 8:5016-5025, 1988), dye incorporation assays (as disclosed by, for example, Mosman, J. Immunol. Meth. 65:55-63, 1983 and Raz et al., Acta Trop. 68:139-147, 1997) or cell counts. Suitable mitogenesis assays measure incorporation of ³H-thymidine into (1) 20% confluent cultures to look for the ability of zvegf3 proteins to further stimulate proliferating cells, and (2) quiescent cells held at confluence for 48 hours to look for the ability of zvegf3 proteins to overcome contact-induced growth inhibition. Suitable dye incorporation assays include measurement of the incorporation of the dye Alamar blue (Raz metal., ibid.) into target cells. See also, Gospodarowicz et al., J. Cell. Biol. 70:395-405, 1976; Ewton and Florini, Endocrinol. 106:577-583, 1980; and Gospodarowicz et al., Proc. Natl. Acad. Sci. USA 86:7311-7315, 1989.

The biological activities of zvegf3 antagonists can be studied in non-human animals by administration of exogenous compounds, by expression of zvegf3 antisense polynucleotides, and by suppression of endogenous zvegf3 expression through knock-out techniques. Viral delivery systems (disclosed above) can be employed. Zvegf3 antagonists can be administered or expressed individually, in combination with other zvegf3 antagonists, or in combination other compounds, including other growth factor antagonists. Test animals are monitored for changes in such parameters as clinical signs, body weight, blood cell counts, clinical chemistry, histopathology, tumor size and progression, and the like.

Animal models that can be used in assessing treatments for hepatocellular carcinoma include transgenic mice (see, e.g., Singh and Kuman, Rev. Med. Virol. 13:243-253, 2003), diethylnitrosamine perfusion in rats (e.g., Wang et al., World J. Gastroenterol. 9:930-935, 2003; Hemmings and Strickland; Cellular Physiology and Biochemistry 12:345-352, 2002), tumor implantation: (e.g., Qian et al.; World J. Gastroenterol. 9:94-98, 2003), the rat multi-organ carcinogenesis model (Hideaki et al., Japanese Journal of Cancer Research 93:1299-1307, 2002) and chronic viral infected woodchucks (Tennant, Clin. Liver Dis. 5:43-68, 2001).

The invention is further illustrated by the following, non-limiting examples.

### EXAMPLES

### Example 1

A human salivary gland library was screened for a full-length clone of zvegf3 by PCR. This library was an arrayed library representing 9.6 X 10⁵ clones made in the vector pZP5x. The vector pZP5x is the same as vector pZP-9 (deposited with American Type Culture Collection, 10801 University Blvd., Manassas, VA under Accession Number 98668), but contains a cytomegalovirus promoter instead of a metallothionein promoter between the Asp718 and BamHI sites. The plasmid thus comprises a dihyrofolate reductase gene under control of the SV40 early promoter and SV40 polyadenylation site, and a cloning site to insert the gene of interest under control of the CMV promoter and the human growth hormone (hGH) gene polyadenylation site. The working plate containing 80 pools of 12,000 colonies each was screened by PCR using oligonucleotide primers ZC19,045 (SEQ ID NO:6) and ZC19,047 (SEQ ID NO:7) with an annealing temperature of 60°C for 35 cycles. There were two strong positives, pools 58 (T-8 F1-F12) and 77 (T-7 H1-H12). The corresponding pools in the transfer plate were then screened by PCR using the same conditions. Two positives were obtained at the transfer level. The positives were T-7 H11 and T-8 F10. 5' RACE reactions were done on the transfer plate pools, and the fragments were sequenced to check zvegf3 sequence and determine if a full-length clone was present. For PCR, oligonucleotide primers ZC12,700 (SEQ ID NO:8) and ZC19,045 (SEQ ID NO:6) were used at an annealing temperature of 61°C for 5 cycles, then 55°C for 30 cycles. Sequencing showed that the pool T-7 H11 had a frameshift. Transfer plate 8 pool F10 sequence appeared to be correct, so this pool of DNA was used in filter lifts.

Pool F10 from transfer plate 8 was plated and filter lifted using nylon membranes (Hybond-N™; Amersham Corporation, Arlington Heights, IL). Approximately 1200 colonies per plate on each of 5 filters were lifted for a total of approximately 6000 colonies. The filters were marked with a hot needle for orientation, then denatured for 6 minutes in 0.5 M NaOH and 1.5 M Tris-HCl, pH 7.2. The filters were then neutralized in 1.5 M NaCl and 0.5 M Tris-HCl, pH 7.2 for 6 minutes. The DNA was affixed to the filters using a UV crosslinker (Stratalinker®, Stratagene, La Jolla, CA) at 1200 joules The filters were prewashed at 65°C: in prewash buffer consisting of 0.25X SSC, 0.25% SDS, and 1mM EDTA. The solution was changed a total of three times over a 45-minute period to remove cell debris. Filters were prehybridized for approximately 3 hours at 65°C in 25 ml of hybridization solution (ExpressHyb™; Clontech Laboratories, Inc., Palo Alto, CA). The probe was generated using an approximately 400-bp fragment produced by digestion of a clone comprising a zvegf3 expressed sequence tag with EcoRI and BgIII followed by gel-purification using a spin column containing a silica gel membrane (QIAquick™ Gel Extraction Kit; Qiagen, Inc., Valencia, CA). The probe was radioactively labeled with ³²P by random priming using a commercially available kit (Rediprime™ II, Amersham Corp., Arlington Heights, IL) and purified using a push column. ExpressHyb™ solution was used for the hybridizing solution for the filters. Hybridization took place overnight at 65°C. Blots were rinsed 2X in 65°C solution 1 (2X SSC, 0.1% SDS), then washed 4 times in solution 1 at 65°C. The filters were exposed to film overnight at -80°C. There were 14 positives on the filters. 85 clones were picked from the positive areas and screened by PCR using oligonucleotide primers ZC19,045 (SEQ ID NO:6) and ZC19,047 (SEQ ID NO:7) and an annealing temperature of 60°C. Thirteen positives were obtained and streaked out for individual clones. Twenty-four colonies were picked and checked by PCR as previously described. Six positives were obtained, two of which were sequenced. Both sequences were the same and full length. The sequence is shown in SEQ ID NO:1.

### Example 2

A PCR panel was screened for mouse zvegf3 DNA. The panel contained 8 cDNA samples from brain, bone marrow, 15-day embryo, testis, salivary gland, placenta, 15-day embryo (Clontech Laboratories), and 17-day embryo (Clontech Laboratories) libraries.

PCR mixtures contained oligonucleotide primers ZC21,222 (SEQ ID NO:9) and ZC21,224 (SEQ ID NO:10). The reaction was run at an annealing temperature of 66°C with an extension time of 2 minutes for a total of 35 cycles using Ex Taq™ DNA polymerase (PanVera, Madison, WI) plus antibody. DNA samples found to be positive for zvegf3 by PCR and confirmed by sequencing included mouse 15-day embryo library total pool cDNA, mouse 15-day embryo (Clontech Laboratories) and 17-day embryo (both obtained from Clontech Laboratories), mouse salivary gland library total pool cDNA, and mouse testis library total pool cDNA. Fragments of about 600 bp from each of the mouse 15-day embryo library total pool cDNA, mouse 15-day embryo cDNA, and mouse 17-day embryo cDNA PCR products were sequenced. Sequence from the mouse 17-day embryo cDNA and mouse 15-day embryo library total pool cDNA products confirmed the fragments to be mouse zvegf3 DNA.

The mouse 15-day embryo library was screened for full-length zvegf3 DNA. This library was an arrayed library representing 9.6 X 10⁵ clones in the pCMV•SPORT 2 vector (Life Technologies, Gaithersburg, MD). The working plate, containing 80 pools of 12,000 colonies each, was screened by PCR using oligonucleotide primers ZC21,223 (SEQ ID NO:11) and ZC21,224 (SEQ ID NO:10) with an annealing temperature of 66°C for 35 cycles. Eighteen positives were obtained. Fragments from four pools (A2, A10, B2, and C4)were sequenced; all were confirmed to encode zvegf3. Additional rounds of screening using the same reaction conditions and pools from the working and source plates identified one positive pool (5D).

Positive colonies were screened by hybridization. Pool 5D from original source plate #5 was plated at about 250 colonies per plate and transferred to nylon membranes (Hybond-N™; Amersham Corporation, Arlington Heights, IL). Five filters were lifted for a total of ∼1250 colonies. The filters were marked with a hot needle for orientation, then denatured for 6 minutes in 0.5 M NaOH and 1.5 M Tris-HCl, pH 7.2. The filters were then neutralized in 1.5 M NaCl and 0.5 M Tris-HCl, pH 7.2 for 6 minutes. The DNA was fixed to the filters using a a UV crosslinker (Stratalinker®, Stratagene, La Jolla, CA) at 1200 joules. A probe was generated by PCR using oligonucleotide primers ZC21,223 (SEQ ID NO:11) and ZC21,224 (SEQ ID NO:10), and a mouse 15-day embryo template at an annealing temperature of 66°C for 35 cycles. The PCR fragment was gel purified using a spin column containing a silica gel membrane (QIAquick™ Gel Extraction Kit; Qiagen, Inc., Valencia, CA). The DNA was radioactively labeled with ³²P using a commercially available kit (Rediprime™ II random-prime labeling system; Amersham Corp., Arlington Heights, IL) according to the manufacturer's specifications. The probe was purified using a commercially available push column (NucTrap® column; Stratagene, La Jolla, CA; see U.S. Patent No. 5,336,412). The filters were prewashed at 65°C in prewash buffer consisting of 0.25X SSC, 0.25% SDS, and 1mM EDTA. The solution was changed a total of three times over a 45-minute period to remove cell debris. Filters were prehybridized overnight at 65°C in 25 ml of a hybridization solution (ExpressHyb™ Hybridization Solution; Clontech Laboratories, Inc., Palo Alto, CA), then hybridized overnight at 65° C in the same solution. Filters were rinsed twice at 65°C in pre-wash buffer (0.25X SSC, 0.25% SDS, and 1mM EDTA), then washed twice in pre-wash buffer at 65°C. Filters were exposed to film for 2 days at -80°C. There were 10 positives on the filters. 3 clones were picked from the positive areas, streaked out, and 15 individual colonies from these three positives were screened by PCR using primers ZC21,223 (SEQ ID NO:11) and ZC21, 334 (SEQ ID NO:12) at an annealing temp of 66°C. Two positives were recovered any sequenced. Both sequences were found to be the same and encoded full-length mouse zvegf3 (SEQ ID NO:4).

The amino acid sequence is highly conserved between mouse and human zvegf3s, with an overall amino acid sequence identity of 87%. The secretory peptide, CUB domain, inter-domain, and growth factor domain have 82%, 92%, 79% and 94% amino acid identity, respectively.

### Example 3

A mammalian cell expression vector for the growth factor domain of zvegf3 was constructed by joining the zvegf3 fragment to a sequence encoding an optimized t-PA secretory signal sequence (U.S. Patent No. 5,641,655) in the linearized pZMP11 vector downstream of the CMV promoter. The plasmid pZMP11 is a mammalian expression vector containing an expression cassette having the CMV immediate early promoter, a consensus intron from the variable region of mouse immunoglobulin heavy chain locus, Kozak sequences, multiple restriction sites for insertion of coding sequences, a stop codon, and a human growth hormone terminator. The plasmid also contains an IRES element from poliovirus, the extracellular domain coding sequence of CD8 truncated at the C-terminal end of the transmembrane domain, an *E. coli* origin of replication, a mammalian selectable marker expression unit having an SV40 promoter, enhancer and origin of replication, a DHFR gene, the SV40 terminator, and the URA3 and CEN-ARS sequences required for selection and replication in *S. cerevisiae.* The resulting vector was designated pZMP11/zv3GF-otPA.

BHK 570 cells were transfected with pZMP11/zv3GF-otPA by liposome-mediated transfection using a 3:1 (w/w) liposome formulation of the polycationic lipid 2,3-dioleyloxy-N-[2(sperminecarboxamido)ethyl]-N,N-dimethyl-1-propaniminium-trifluoroacetate and the neutral lipid dioleoyl phosphatidylethanolamine in membrane-filtered water (Lipofectamine™; Life Technologies) and cultured according to conventional procedures.

BHK cell-conditioned media was adjusted to 20 mM MES at pH 5.5. A column of cation exchange resin (Poros® HS 50; PerSeptive Biosystems, Framingham, MA) (2-cm diameter; 50 ml bed volume) was equilibrated in 20 mM MES, 150 mM NaCl, pH 5.5. The adjusted media was pumped into the column at 20 ml/minute. When loading was completed, the column was washed, first in 20 mM MES, 150 mM NaCl, pH 5.5, then with the same composition buffer at pH 6.0. Once the baseline was back to zero absorbance, the column was eluted with a 10-column-volume gradient to 20 mM MES, 1 M NaCl, pH 6.0. The zvegf3 growth factor domain eluted at 25 to 50 mS conductivity during the evolving gradient. Reducing SDS-PAGE revealed a distinct band at 20 kD, which was confirmed as zvegf3 by Western blotting. This material was pooled and prepared for loading to a tandem column array comprising a strong anion exchange resin (Poros® HQ 50; PerSeptive Biosystems) followed by an immobilized metal (nickel) affinity column in series. The system of columns was equilibrated in 20 mM MOPS buffer at pH 7.0. The vegf3 pool was in-line diluted at 1:10 (v/v) with the MOPS equilibration buffer while loading. After loading was completed the column series was washed with 20 mM MOPS pH 7.0 buffer until baseline absorbance was obtained. The nickel column was then disconnected fron the anion exchanger and washed with 20 mM MOPS pH 7.0 containing 150 mM NaCl. The column was then eluted with a 1-column-volume gradient between the last washing buffer and the same buffer containing 20 mM imidazole at pH 7.0. The fractions containing the zvegf3 growth factor domain were pooled and concentrated using a 5 kD cuttoff membrane in preparation for buffer exchange and polishing on a size exclusion column equilibrated in PBS.

### Example 4

To make transgenic animals expressing zvegf3 genes requires adult, fertile males (studs) (B6C3f1, 2-8 months of age (Taconic Farms, Germantown, NY)), vasectomized males (duds) (B6D2f1, 2-8 months, (Taconic Farms)), prepubescent fertile females (donors) (B6C3f1, 4-5 weeks, (Taconic Farms)) and adult fertile females (recipients) (B6D2f1, 2-4 months, (Taconic Farms)).

The donors are acclimated for 1 week, then injected with approximately 8 IU/mouse of Pregnant Mare's Serum gonadotrophin (Sigma Chemical Co., St. Louis, MO) I.P., and 46-47 hours later, 8 IU/mouse of human Chorionic Gonadotropin (hCG (Sigma Chemical Co.)) I.P. to induce superovulation. Donors are mated with studs subsequent to hormone injections. Ovulation generally occurs within 13 hours of hCG injection. Copulation is confirmed by the presence of a vaginal plug the morning following mating.

Fertilized eggs are collected under a surgical scope (Leica MZ12 Stereo Microscope, Leica, Wetzlar, Germany). The oviducts are collected and eggs are released into urinanalysis slides containing hyaluronidase (Sigma Chemical Co.). Eggs are washed once in hyaluronidase, and twice in Whitten's W640 medium (Table 2; all reagents available from Sigma Chemical Co.) that has been incubated with 5% CO₂, 5% O₂, and 90% N₂ at 37°C. The eggs are stored in a 37°C/5% CO₂ incubator until microinjection.

**Table 2**

| | mgs/200 ml | mgs/500 ml |
|---|---|---|
| NaCl | 1280 | 3200 |
| KCl | 72 | 180 |
| KH₂PO₄ | 32 | 80 |
| MgSO_{4·}7H₂O | 60 | 150 |
| Glucose | 200 | 500 |
| Ca²⁺ Lactate | 106 | 265 |
| Benzylpenicillin | 15 | 37.5 |
| Streptomycin SO₄ | 10 | 25 |
| NaHCO₃ | 380 | 950 |
| Na Pyruvate | 5 | 12.5 |
| H₂O | 200 ml | 500ml |
| 500 mM EDTA | 100 µl | 250 µl |
| 5% Phenol Red | 200 µl | 500 µl |
| BSA | 600 | 1500 |

Zvegf3 cDNA is inserted into the expression vector pHB12-8. Vector pHB12-8 was derived from p2999B4 (Palmiter et al., Mol. Cell Biol. 13:5266-5275, 1993) by insertion of a rat insulin II intron (ca. 200 bp) and polylinker (Fse I/Pme I/Asc 1) into the Nru I site. The vector comprises a mouse metallothionein (MT-1) promoter (ca. 750 bp) and human growth hormone (hGH) untranslated region and polyadenylation signal (ca. 650 bp) flanked by 10 kb of MT-1 5' flanking sequence and 7 kb of MT-1 3' flanking sequence. The cDNA is inserted between the insulin II and hGH sequences.

10-20 micrograms of plasmid DNA is linearized, gel-purified, and resuspended in 10 mM Tris pH 7.4, 0.25 mM EDTA pH 8.0, at a final concentration of 5-10 nanograms per microliter for microinjection.

Plasmid DNA is microinjected into harvested eggs contained in a drop of W640 medium overlaid by warm, CO₂-equilibrate mineral oil. The DNA is drawn into an injection needle (pulled from a 0.75 mm ID, 1 mm OD borosilicate glass capillary) and injected into individual eggs. Each egg is penetrated with the injection needle into one or both of the haploid pronuclei.

Picoliters of DNA are injected into the pronuclei, and the injection needle is withdrawn without coming into contact with the nucleoli. The procedure is repeated until all the eggs are injected. Successfully microinjected eggs are transferred into an organ tissue-culture dish with pregassed W640 medium for storage overnight in a 37°C/5% CO₂ incubator.

The following day, 2-cell embryos are transferred into pseudopregnant recipients. The recipients are identified by the presence of copulation plugs, after copulating with vasectomized duds. Recipients are anesthetized and shaved on the dorsal left side and transferred to a surgical microscope. A small incision is made in the skin and through the muscle wall in the middle of the abdominal area outlined by the ribcage, the saddle, and the hind leg, midway between knee and spleen. The reproductive organs are exteriorized onto a small surgical drape. The fat pad is stretched out over the surgical drape, and a baby serrefine (Roboz, Rockville, MD) is attached to the fat pad and left hanging over the back of the mouse, preventing the organs from sliding back in. With a fine transfer pipette containing mineral oil followed by alternating W640 and air bubbles, 12-17 healthy 2-cell embryos from the previous day's injection are transferred into the recipient. The swollen ampulla is located, and holding the oviduct between the ampulla and the bursa, a nick in the oviduct is made with a 28 g needle close to the bursa, making sure not to tear the ampulla or the bursa. The pipette is transferred into the nick in the oviduct, and the embryos are blown in, allowing the first air bubble to escape the pipette. The fat pad is gently pushed into the peritoneum, and the reproductive organs are allowed to slide in. The peritoneal wall is closed with one suture, and the skin is closed with a wound clip. The mice recuperate on a 37°C slide warmer for a minimum of 4 hours.

The recipients are returned to cages in pairs, and allowed 19-21 days gestation. After birth, 19-21 days postpartum is allowed before weaning. The weanlings are sexed and placed into separate sex cages, and a 0.5 cm biopsy (used for genotyping) is snipped off the tail with clean scissors.

Genomic DNA is prepared from the tail snips using a commercially available kit (DNeasy™ 96 Tissue Kit; Qiagen, Valencia, CA) following the manufacturer's instructions. Genomic DNA is analyzed by PCR using primers designed to the human growth hormone (hGH) 3' UTR portion of the transgenic vector. The use of a region unique to the human sequence (identified from an alignment of the human and mouse growth hormone 3' UTR DNA sequences) ensures that the PCR reaction does not amplify the mouse sequence. Primers ZC17,251 (SEQ ID NO:13) and ZC17,252 (SEQ ID NO:14) amplify a 368-base-pair fragment of hGH. In addition, primers ZC17,156 (SEQ ID NO:15) and ZC17,157 (SEQ ID NO:16), which hybridize to vector sequences and amplify the cDNA insert; may be used along with the hGH primers. In these experiments, DNA from animals positive for the transgene will generate two bands, a 368-base-pair band corresponding to the hGH 3' UTR fragment and a band of variable size corresponding to the cDNA insert.

Once animals are confirmed to be transgenic (TG), they are back-crossed into an inbred strain by placing a TG female with a wild-type male, or a TG male with one or two wild-type female(s). As pups are born and weaned, the sexes are separated, and their tails snipped for genotyping.

To check for expression of a transgene in a live animal, a partial hepatectomy is performed. A surgical prep is made of the upper abdomen directly below the xiphoid process. Using sterile technique, a small 1.5-2 cm incision is made below the sternum, and the left lateral lobe of the liver is exteriorized. Using 4-0 silk, a tie is made around the lower lobe securing it outside the body cavity. An atraumatic clamp is used to hold the tie while a second loop of absorbable Dexon (American Cyanamid, Wayne, N.J.) is placed proximal to the first tie. A distal cut is made from the Dexon tie, and approximately 100 mg of the excised liver tissue is placed in a sterile petri dish. The excised liver section is transferred to a 14-ml polypropylene round bottom tube, snap-frozen in liquid nitrogen, and stored on dry ice. The surgical site is closed with suture and wound clips, and the animal's cage is placed on a 37°C heating pad for 24 hours post-operatively. The animal is checked daily post-operatively, and the wound clips are removed 7-10 days after surgery.

Analysis of the mRNA expression level of each transgene is done using an RNA solution hybridization assay or real-time PCR on commercially available PCR equipment (ABI Prism^{™} 7700; PE Applied Biosystems, Inc., Foster City, CA) following the manufacturer's instructions.

An adenovirus vector was prepared using a liver-specific albumin gene enhancer and basal promoter (designated "AEO promoter"). The albumin promoter construct (designated pAEO) was constructed by inserting a 2.2 kb Not1/EcoRV fragment from pALBdelta2L (Pinkert et al., Genes Dev. 1:268-276, 1987) and an 850 bp NruI/Not1 DNA segment comprising the rat insulin II intron, an FseI/PmeI/AscI polylinker, and the human growth hormone poly A sequence into a commercially available phagemid vector (pBluescript® KS(+); Stratagene, La Jolla, CA). For microinjection, the plasmid is digested with Not1 to liberate the expression cassette.

An additional adenovirus vector was constructed using an epithelial cell-specific keratin gene (K14) promoter (Vassar et al., Proc. Natl. Acad. Sci. USA 86:1563-1567, 1989). The 1038-bp open reading frame encoding full-length human zvegf3 was amplified by PCR so as to introduce an optimized initiation codon and flanking 5' *Pme*I and 3' *Asc*I sites using the primers: ZC20,180 (SEQ ID NO:17) and ZC20,181 (SEQ ID NO:18). The resulting *Pme*I/*Asc*I fragment was subcloned into the polylinker of pKF0114, a basal keratinocyte-restricted transgenic vector comprising the human keratin 14 (K14) promoter (an approximately 2.3 Kb fragment amplified from human genomic DNA [obtained from Clontech Laboratories, Inc.] based on the sequence of Staggers et al., "Sequence of the promoter for the epidermal keratin gene, K14", GenBank accession #U11076, 1994), followed by a heterologous intron (a 294-bp *BstX*Il*Pst*I fragment from pIRES1hyg (Clontech Laboratories, Inc.; see, Huang and Gorman, Nucleic Acids Res. 18:937-947, 1990), a *Pme*I*lAsc*I polylinker, and the human growth hormone gene polyadenylation signal (a 627 bp *Sma*I*lEco*RI fragment; see, Seeburg, DNA 1:239-249, 1982). The transgene insert was separated from the plasmid backbone by *Not*I digestion and agarose gel purification, and fertilized ova from matings of B6C3F1Tac mice or inbred FVB/NTac mice were microinjected and implanted into pseudopregnant females essentially as described by Malik et al., *Molec. Cell. Biol.* 15:2349-2358, 1995. Transgenic founders were identified by PCR on genomic tail DNA using primers specific for the human growth hormone poly A signal (ZC 17,252, SEQ ID NO:14; and ZC17,251, SEQ ID NO:13) to amplify a 368-bp diagnostic product. Transgenic lines were initiated by breeding founders with C57BL/6Tac or FVB/NTac mice.

Transgenic mice were generated essentially as disclosed above using MT-1, K14, and AEO promoters. Four MT-1/zvegf3 transgenic mice were generated. In one animal (female) approximately 800 molecules zvegf3 mRNA/cell were produced in the liver after zinc induction. This animal had enlargement of the liver and spleen. Also observed were proliferation of hepatic sinusoidal cells and extra-medullary hematopoiesis. One K14/zvegf3 transgenic mouse (female) showed a low level of expression with low body weight, low hematocrit, and low platelet count. One AEO/zvegf3 transgenic mouse (male) with a low level of expression exhibited liver sinusoidal cell proliferation.

Histological analysis was carried out on livers from N1 transgenic mice overexpressing full-length zvegf3 from the AEO promoter and nontransgenic littermate controls at 8, 16, 22, and 33 weeks of age. Similar changes were seen in male and female animals. H & E and trichrome stains indicated a definite increase in number of liver sinusoidal (stellate) cells and increased perisinusoidal extracellular matrix (ECM) deposition at 8 weeks of age. There was a persistent increase in number of stellate cells and in the amount and thickness of perisinusoidal ECM as well as some perivenular ECM deposition by 16 weeks. At 22 weeks similar changes were seen, but with an increase in incidence and severity of perivenular fibrosis (steato fibrosis). Changes similar to those at 16 and 22 weeks were observed at 33 weeks, however some animals had fibrotic, banding and multiple, encapsulated, areas in which hepatocytes appeared enlarged and vacuolated. These areas tended to be surrounded by a fibrous tissue capsule, and the hepatocytes within these areas appeared normal. These changes were consistent with early cirrhosis.

Similar changes were seen in 8-week-old N2 mice and in breeder males sacrificed at approximately 32-34 weeks.

### Example 5

Five AEO human zvegf3 transgenic mice (N7 generation) were sacrificed and necropsied, and tissues were collected in 10% buffered formalin. Additional liver samples were fixed in Carnoy's and zinc tris fixatives for immunohistochemistry. Individual animal reports for each of the five mice are shown below.

### Animal No. 30054 (male, 53 weeks of age)

At necropsy, the liver appeared diffusely large and swollen with rounded edges, and the left lateral lobe had a distinctly nodular appearance. Microscopically, the major findings were in the liver and included sinusoidal cell proliferation, nodular hyperplasia, and hepatocellular adenoma. In addition, the hepatocytes were vacuolated, especially around the central vein, and there were some mild or slight focal mononuclear cell infiltrates and bile duct hyperplasia. Slight to moderate mononuclear cell infiltrates were also observed in the lung, kidney, pancreas, and salivary gland. Lymphoreticular hyperplasia was observed in the thymus, spleen, and bone marrow, the latter being nodular in appearance.

### Animal No. 30237 (male, 52 weeks of age)

At necropsy, the larger lobes of the liver contained large cysts and multiple nodules. In addition, the spleen was observed to be 2-3 times its normal size. Microscopically, the liver was the most severely affected organ examined; findings included moderate sinusoidal cell proliferation, nodular/hepatocellular hyperplasia, and severe (diffuse) vascular dilatation. One section had an area of thrombosis and coagulative necrosis, the latter indicative of infarction possibly caused by the thrombosis. The spleen had moderate lymphoreticular hyperplasia, which correlated with the gross observation of being enlarged.

### Animal No. 30031 (female, 53 weeks of age)

Gross observations at necropsy included an enlarged liver with rounded edges containing multiple nodules throughout. The right and left lateral lobes were especially prominent, and the blood vessels in the right lateral lobe were prominent. The small median lobe was tan, and the spleen appeared slightly enlarged. Microscopically, one section of the liver contained an area of hepatocellular adenoma, and there were multiple areas with nodular hepatocellular hyperplasia. The liver also had moderate sinusoidal cell proliferation, sinusoidal dilatation, and focal vascular dilatation. Findings in other tissues included cystic endometrial hyperplasia of the uterus; mononuclear cell infiltrate in the lung (peribronchial), kidney, pancreas, and salivary gland; and lymphoreticular hyperplasia in the thymus, spleen and lymph node. The kidneys had a mild glomerulopathy that was characterized as increased hyaline material in the glomerular tuft and/or increased mesangium. There were also focal areas of tubular regeneration and tubules dilated with proteinaceous material.

### Animal No. 30032 (female, 53 weeks of age)

At necropsy the liver appeared pale and contained multiple nodules throughout. Microscopically, the liver had multiple areas of variable size of nodular (hepatocellular) hyperplasia that correlated with the gross observation. The liver also had moderate sinusoidal cell proliferation and mononuclear cell infiltrate, and mild or slight sinusoidal and vascular dilatation, some with evidence of perivenular fibrosis. The kidneys had a moderate glomerulopathy that was characterized as increased hyaline material in the glomerular tuft and/or increased mesangium. There were also focal areas of tubular regeneration and tubules dilated with proteinaceous material. Findings in other tissues included cystic endometrial hyperplasia of the uterus; mononuclear cell infiltrate in the lung (peribronchial), kidney, pancreas, and salivary gland; and lymphoreticular hyperplasia in the spleen and lymph node.

### Animal No. 30033 (female. 53 weeks of age)

At necropsy, the liver was observed to be enlarged with prominent blood vessels, multiple nodules of variable size, and focal cyst formation. The right lateral lobe had a papillary mass attached to its surface via a narrow stalk, and the caudal lobe was observed to be swollen and dark red. Microscopically, the liver had multiple areas of nodular (hepatocellular) hyperplasia and moderate sinusoidal cell proliferation with a focal area of perivenular fibrosis. Other findings in this animal included mononuclear cell infiltrate in the liver, lung (peribronchial), kidney, pancreas, and salivary gland, and lymphoreticular hyperplasia in the thymus, spleen and lymph node. The kidneys had mild or slight glomerulopathy similar to that described above but lacked any obvious tubular changes.

In summary, all five animals had similar changes in the tissues examined microscopically. The more prominent findings were in the liver, which were phenotypical of this construct. There was a moderate increase in the number or hyperplasia of sinusoidal cells, which, although not identified positively, were thought to be hepatic stellate cells (HSC). Immunohistochemistry and special stains revealed that, although these cells had no evidence of alpha smooth muscle actin (an indicator of HSC activation), there was a significant increase in the amount of intra-sinusoidal collagen, the latter visualized by Sirius Red staining. There was some evidence of perivenular fibrosis in a couple of animals. Increased collagen deposition may have initiated and/or contributed to the hepatocellular hyperplasia and nodule formation, which in turn resulted in tumor formation as represented by the hepatocellular adenomas observed in some livers. All findings in the remaining tissues examined from these animals, such as the mononuclear cell infiltrates in various organs, lymphoreticular hyperplasia in the lymphoid tissues, and renal changes, were reflective of the general declining health of the individual animal and were not thought to be directly related to the transgene. Some findings are common in most mouse strains at this age.

### Example 6

Two male transgenic mice, N4 generation, were sacrificed at 52 weeks of age and necropsied, and a routine physioscreen was conducted. Microscopically, the liver was the primary target organ, with a multitude of changes that included telangiectasis, vascular dilatation, sinusoidal cell proliferation, nodular hepatocytic hyperplasia, and sinusoidal fibrosis. In at least one of the two animals, the liver also had areas of hemorrhage, infarction, bile duct hyperplasia, cyst formation, and lymphoid cell infiltrates. The microscopic observations in the remaining tissues examined were considered incidental findings common in one-year old mice.

The progressive hepatic fibrosis in these two animals resulted in the disruption of the architecture of the vascular system causing severe hypertension as evidenced by the dilated vessels and the telangiectasis within the sinusoids. Due to the extensive loss of normal hepatic parenchyma in the areas of telangiectasis, the severity of the sinusoidal cell proliferation was difficult to assess, although there were areas of obvious increased numbers of these cells in the absence of hepatocyte loss.

### Example 7

A single male transgenic AEO/zvegf3 mouse, N4 generation, was sacrificed at 57 weeks of age and necropsied, and a routine physioscreen was conducted. At necropsy the liver was noted to be misshapen, dark, and nodular. Microscopically, the liver had areas of moderate diffuse perisinusoidal (stellate) cell hyperplasia as well as moderate sinusoidal dilation, myxomatous change, perivascular fibrosis, and nodular hepatocellular hyperplasia. Also present in one or more of the sections examined were a cyst, a hepatocellular adenoma, and a focal area of necrosis. The latter finding was believed to represent an area of infarction that was possibly associated with the fibrosis and areas of myxomatous change. All of these changes were considered typical of progressive and chronic hepatic fibrosis and were similar to that observed in other transgenic mice of this construct and age. All remaining microscopic observations in this animal were considered incidental findings and unrelated to the transgene.

### Example 8

Four AEO/zvegf3 transgenic mice (N4) were sacrificed at approximately 62 weeks of age and necropsied, and tissues were collected and preserved in 10% BNF (buffered neutral formalin). At necropsy, the liver in each of these mice was observed to be enlarged and fibrotic in appearance. All tissues were trimmed and processed, slides were prepared, and sections were stained with hematoxylin and eosin for routine microscopic examination. In addition, sections of liver were stained with Masson's trichrome and Sirius Red, and immunohistochemistry (IHC) was done for the detection of α smooth muscle actin, desmin, and Type I collagen.

### Animal No. 24811- male

The most prominent microscopic findings were in the liver, foremost being severe diffuse sinusoidal cell hyperplasia/proliferation with multifocal areas of myxomatous-like matrix accumulation, the latter characterized as accumulation of acidic mucopolysaccharide-like material surrounding spindle shaped cells that were similar in appearance to the adjacent proliferating sinusoidal (possibly stellate) cells. There was an apparent dilation of all blood vessels, especially the central veins, as well as multifocal areas of sinusoidal dilation and telangiectasis. The latter changes were often accompanied by increased numbers of plump, endothelial-like cells that lined the walls of these dilated areas. Some areas within the sections had an obvious increase in fibrosis and suggestion of hepatic nodule formation, the latter more readily seen with the trichrome and Sirius Red stained sections. Immunohistochemistry (IHC) demonstrated increased alpha smooth muscle actin (SM actin) staining, primarily associated with the areas of sinusoidal dilatation and endothelial-like cell proliferation, and was highly suggestive of possible angiogenesis. There was also a correlating increase in the staining for desmin. Type I collagen staining, correlated closely with the fibrosis; collagen was most abundant in the fibrosis associated with possible hepatic nodule formation. In other tissues, prominent findings included perivascular lymphoid hyperplasia in the lung, a cortical cyst in one kidney, multifocal lymphoid infiltrate, and diffuse glomerulopathy in both kidneys. The glomerulopathy was characterized by an increase in the overall size of the glomeruli as well as an increased cellularity. Some of the affected glomeruli contained eosinophilic droplets of variable size that were thought to represent protein. (These findings in the lung and kidney are not uncommon in aged mice and may possibly be secondary to the liver pathology.) The mesenteric lymph nodes were completely replaced by a neoplasm that was composed of spindle-shaped cells embedded in a fibrous appearing matrix. This tumor was diagnosed as a spindle cell sarcoma and considered an incidental finding and unrelated to the transgene. All microscopic changes observed in the remaining tissues were also considered incidental findings.

### Animal No. 24957 - male

The microscopic findings in this animal were similar to those observed in Animal No. 24811 and again, the most prominent findings were in the liver. However, the main difference was the presence of rather large neoplasms in two or more lobes of the liver that were characterized as being composed of numerous plump, endothelial-like cells that lined or tended to pile up in prominent dilated sinusoids and areas of telangiectases. This tumor was diagnosed as a hemangioendothelioma. There were also focal areas of necrosis, possibly infarction, associated with the neoplasm. Except for the absence of myxomatous areas, the remaining liver had changes similar to those described previously, i.e., sinusoidal cell hyperplasia, nodular hepatocellular hyperplasia, lymphoid infiltrate, vascular dilatation, and fibrosis. In the IHC-stained sections, SM actin and desmin were most prominent within the neoplasm and were possibly reflective of angiogenesis. The Type I collagen was not as prominent within the tumor but was more prominent in the areas of fibrosis and nodular hyperplasia. Other prominent changes in the kidney and lung were similar in both distribution and severity to those described above for Animal No. 24811. All microscopic changes observed in the remaining tissues were also considered incidental findings and unrelated to the transgene.

### Animal No. 26015 - male

The microscopic findings in the liver from this animal were very similar to those observed in Animals Nos. 24811 and 24957 with the exception of a large mass in one lobe. This neoplasm was characterized as a thinly encapsulated mass composed of cords of large, vacuolated hepatocytes separated by dilated sinusoids with focal areas of necrosis due to infarction. This neoplasm was characteristic of a hepatocellular adenoma. There were minimal amounts of SM actin, desmin, and Type I collagen within the tumor, but increased amounts of all three of these were present in the other sections of the liver, the latter two most prominent in the areas of increased sinusoidal cell hyperplasia and fibrosis. Microscopic changes in the kidney and lung were also similar to those observed in the two previous animals. All other microscopic changes observed in the remaining tissues were considered incidental findings and not directly related to the transgene.

### Animal No. 24819 - female

The microscopic findings in the tissues examined from this animal were very similar to those observed in the three male mice described above. The liver had severe diffuse sinusoidal hyperplasia, fibrosis, and hepatocellular nodular hyperplasia as well as vascular and sinusoidal dilatation, bile duct hyperplasia, and focal areas of myxomatous-like matrix accumulation. There was an increase in the amount of fibrosis throughout the sections of liver examined, which was most prominent around areas of hepatocytic nodule formation. Increased staining for desmin and Type I collagen was also prominent in these areas and was closely associated with the fibrosis. There were some focal areas of increased staining for SM actin, but these were primarily associated with areas of sinusoidal dilatation and telangiectasis, and possibly represented angiogenesis. The microscopic changes observed in the kidney and lung were also similar to those previously described above. However, the lung in this animal also had a moderate chronic pleuritis characterized primarily as pleura fibrosis. This latter observation as well as the changes observed in the remaining tissues examined were considered incidental findings and unrelated to the transgene.

The microscopic changes observed in the livers from these four transgenic mice may represent the end stage of a chronic progressive fibrosis. The hepatocytic nodule and tumor formation is similar to that ascribed to hepatic fibrosis and cirrhosis in man. Further, the impact of the resulting fibrosis and the sinusoidal cell accumulation on the vascular system is evident from the vascular and sinusoidal dilatation as well as the development of the vascular neoplasms and associated preneoplastic changes. Interestingly, the results of the IHC staining for α smooth muscle actin, which appeared to be primarily associated with the angiogenic changes and not the sinusoidal cells or the fibrotic areas, would suggest that the cells involved in matrix and collagen production may have differentiated into more specialized cells that have lost their actin and therefore can no longer be termed myofibroblasts.

As disclosed in Examples 5-8, twelve AEO human zvegf3. transgenic mice between 52 and 62 weeks of age were sacrificed and necropsied. At necropsy, selected tissues were collected and processed for microscopic evaluation with special attention given to the pathology of the liver. At necropsy, the liver from each of these animals was observed to be variably enlarged, swollen, mis-shaped, abnormally dark or light colored, fibrotic, nodular, and/or containing cysts. Microscopically, the variable but consistent, prominent findings in the liver were phenotypical of this transgenic construct. Although not present in every animal, one or more of the following hepatic changes were observed: increased numbers or hyperplasia of perisinusoidal (stellate) cells, sinusoidal and vascular dilatation, telangiectasis, cyst formation, perivenular and intra-sinusoidal fibrosis, nodular hepatocellular hyperplasia, and hepatocellular adenoma. Occasionally associated with the fibrosis and the proliferating perisinusoidal cells was a myxomatous change characterized by the presence of spindle-shaped cells embedded in a mucinous matrix. One animal had a hepatic tumor composed of plump, endothelial-like cells associated with areas of vascular and sinusoidal dilatation. This tumor was characterized as a hemangioendothelioma, and its formation was possibly related to the fibrosis and associated vascular changes. Some of the areas of nodular hyperplasia and adenoma formation frequently had areas of necrosis that was attributed to infarction.

The gross and correlating microscopic changes observed in the livers from these older transgenic mice were typical of the end stage of a chronic, progressive fibrosis. The progressive increase in collagen deposition and subsequent fibrosis observed is similar to hepatic fibrosis and cirrhosis in man, and is commonly thought to contribute to the nodular hepatocellular hyperplasia and tumor formation, including a progression from hyperplasia to adenoma to carcinoma.

### Example 9

For construction of adenovirus vectors, the protein coding region of human zvegf3 was amplified by PCR using primers that added PmeI and AscI restriction sties at the 5' and 3' termini, respectively. PCR primers ZC20,180 (SEQ ID NO:17) and ZC20,181 (SEQ ID NO:18) were used with a full-length zvegf3 cDNA template in a PCR reaction as follows: incubation at 95°C for 5 minutes; followed by 15 cycles at 95°C for 1 min., 61°C for 1 min., and 72°C for 1.5 min.; followed by 72°C for 7 min.; followed by a 4°C soak. The reaction product was loaded onto a 1.2 % low-melting-temperature agarose gel in TAE buffer (0.04 M Tris-acetate, 0.001 M EDTA). The zvegf3 PCR product was excised from the gel and purified using a commercially available kit comprising a silica gel mambrane spin column (QIAquick™ PCR Purification Kit and gel cleanup kit; Qiagen, Inc.) as per kit instructions. The zvegf3 product was then digested with PmeI and AscI, phenol/chloroform extracted, EtOH precipitated, and rehydrated in 20 ml TE (Tris/EDTA pH 8). The 1038 bp zvegf3 fragment was then ligated into the PmeI-AscI sites of the transgenic vector TG12-8 (also known as pHB12-8; see Example 4) and transformed into *E. coli* DH10B™ competent cells by electroporation. Clones containing zvegf3 were identified by plasmid DNA miniprep followed by digestion with PmeI and AscI. A positive clone was sequenced to insure that there were no deletions or other anomalies in the construct. The sequence of zvegf3 cDNA was confirmed.

DNA was prepared using a commercially available kit (Maxi Kit, Qiagen, Inc.), and the 1038bp zvegf3 cDNA was released from the pTG12-8 vector using Pmel and AscI enzymes. The cDNA was isolated on a 1% low melting temperature agarose gel and was excised from the gel. The gel slice was melted at 70°C, and the DNA was extracted twice with an equal volume of Tris-buffered phenol and precipitated with EtOH. The DNA was resuspended in 10 µl H₂O.

The zvegf3 cDNA was cloned into the EcoRV-AscI sites of a modified pAdTrack-CMV (He, T-C. et al., Proc. Natl. Acad. Sci. USA 95:2509-2514, 1998). This construct contains the green fluorescent protein (GFP) marker gene. The CMV promoter driving GFP expression was replaced with the SV40 promoter, and the SV40 polyadenylation signal was replaced with the human growth hormone polyadenylation signal. In addition, the native polylinker was replaced with FseI, EcoRV, and AscI sites. This modified form of pAdTrack-CMV was named pZyTrack. Ligation was performed using a commercially available DNA ligation and screening kit (Fast-Link™ kit; Epicentre Technologies, Madison, WI). Clones containing zvegf3 were identified by digestion of mini prep DNA with FseI and AscI. In order to linearize the plasmid, approximately 5 µg of the resulting pZyTrack zvegf3 plasmid was digested with PmeI. Approximately 1 µg of the linearized plasmid was cotransformed with 200 ng of supercoiled pAdEasy (He et al., *ibid.*) into *E. coli* BJ5183 cells (He et al., *ibid.*). The co-transformation was done using commercially available electroporation equipment (Gene Pulser®; Bio-Rad Laboratories, Hercules, CA) at 2.5 kV, 200 ohms and 25 µFa. The entire co-transformation mixture was plated on 4 LB plates containing 25 µg/ml kanamycin. The smallest colonies were picked and expanded in LB/kanamycin, and recombinant adenovirus DNA was identified by standard DNA miniprep procedures. Digestion of the recombinant adenovirus DNA with FseI and AscI confirmed the presence of the zvegf3 insert. The recombinant adenovirus miniprep DNA was transformed into *E., coli* DH10B™ competent cells, and DNA was prepared using a commercially available, DNA purification kit (obtained from Qiagen, Inc.) according to kit instructions.

Approximately 5 µg of recombinant adenoviral DNA was digested with PacI enzyme (New England Biolabs) for 3 hours at 37°C in a reaction volume of 100 µl containing 20-30U of PacI. The digested DNA was extracted twice with an equal volume of phenol/chloroform and precipitated with ethanol. The DNA pellet was resuspended in 10 µl distilled water. A T25 flask of QBI-293A cells (Quantum Biotechnologies, Inc. Montreal, Qc. Canada), inoculated the day before and grown to 60-70% confluence, were transfected with the PacI-digested DNA. The PacI-digested DNA was diluted up to a total volume of 50 µl with sterile HBS (150 mM NaCl, 20 mM HEPES). In a separate tube, 20 µl of 1 mg/ml N-[1-(2,3-Dioleoyloxy)propyl]-N,N,N-trimethyl-ammonium salts (DOTAP) (Boehringer Mannheim, Indianapolis, IN) was diluted to a total volume of 100 µl with HBS. The DNA was added to the DOTAP, mixed gently by pipeting up and down, and left at room temperature for 15 minutes. The media was removed from the 293A cells and washed with 5 ml serum-free minimum essential medium (MEM) alpha containing 1 mM sodium pyruvate, 0.1 mM MEM non-essential amino acids, and 25mM HEPES buffer (reagents obtained from Life Technologies, Gaithersburg, MD). 5 ml of serum-free MEM was added, and the cells were held at 37°C. The DNA/lipid mixture was added drop-wise to the flask of cells, mixed gently, and incubated at 37°C for 4 hours. The media containing the DNA/lipid mixture was then aspirated off and replaced with 5 ml complete MEM containing 5% fetal bovine serum. The transfected cells were monitored for GFP expression and formation of foci (viral plaques).

Seven days after transfection of 293A cells with the recombinant adenoviral DNA, the cells expressed GFP and started to form foci. The crude viral lysate was collected using a cell scraper to *collect* all of the 293A cells. The lysate was transferred to a 50-ml conical tube. To release most of the virus particles from the cells, three freeze/thaw cycles were done in a dry ice/ethanol bath and a 37° waterbath.

The crude lysate was amplified (Primary (1°) amplification) to obtain a working stock of zvegf3 rAdV lysate. Ten 10-cm plates of nearly confluent (80-90%) 293A cells were set up 20 hours previously, then 200 µl of crude rAdV lysate was added to each 10-cm plate, and the plates were monitored for 48 to 72 hours looking for cytopathic effect (CPE) under the white light microscope and expression of GFP under the fluorescent microscope. When all of the cells showed CPE this 1° stock lysate was collected, and freeze/thaw cycles were performed as described above.

Secondary (2°) amplification of zvegf3 rAdV was obtained as follows: Twenty 15-cm tissue cultured dishes of 293A cells were prepared so that the, cells were 80-90% confluent. All but 20 ml of 5% MEM media was removed, and each dish was inoculated with 300-500 µl of the 1° amplified rAdv lysate. After 48 hours the cells were lysed from virus production, the lysate was collected into 250-ml polypropylenes centrifuge bottles, and the rAdV was purified.

NP-40 detergent was added to a final concentration of 0.5% to the bottles of crude lysate in order to lyse all cells. Bottles were placed on a rotating platform for 10 minutes agitating as fast as possible without the bottles falling over. The debris was pelleted by centrifugation at 20,000 X G for 15 minutes. The supernatant was transferred to 250-ml polycarbonate centrifuge bottles, and 0.5 volume of 20% PEG8000/2.5 M NaCl solution was added. The bottles were shaken overnight on ice. The bottles were centrifuged at 20,000 X G for 15 minutes, and the supernatant was discarded into a bleach solution. Using a sterile cell scraper, the white, virus/PEG precipitate from 2 bottles was resuspended in 2.5 ml PBS. The resulting virus solution was placed in 2-ml microcentrifuge tubes and centrifuged at 14,000 X G in the microcentrifuge for 10 minutes to remove any additional cell debris. The supernatant from the 2-ml microcentrifuge tubes was transferred into a 15-ml polypropylene snapcap tube and adjusted to a density of 1.34 g/ml with CsCl. The volume of the virus solution was estimated, and 0.55 g/ml of CsCl was added. The CsCl was dissolved, and 1 ml of this solution weighed 1.34 g. The solution was transferred to 3.2-ml, polycarbonate, thick-walled centrifuge tubes and spun at 348,000 X G for 3-4 hours at 25°C. The virus formed a white band. Using wide-bore pipette tips, the virus band was collected.

The virus recovered from the gradient had a large amount of CsCl, which had to be removed before the virus was used on cells. Commercially available ionexchange columns (PD-10 columns prepacked with Sephadex® G-25M; Pharmacia Biotech, Piscataway, NJ) were used to desalt the virus preparation. The column was equilibrated with 20 ml of PBS. The virus was loaded onto and allowed to run into the column. 5 ml of PBS was added to the column, and fractions of 8-10 drops were collected. The optical density of a 1:50 dilution of each fraction was determined at 260 nm on a spectrophotometer. A clear absorbance peak was present between fractions 7-12. Peak fractions were pooled, and the optical density (OD) of a 1:25 dilution was determined. OD was converted to virus concentration using the formula: (OD at 260nm)(25)(1.1 x 10¹²) = virions/ml. The OD of a 1:25 dilution of the zvegf3 rAdV was 0.145, giving a virus concentration of 4 X 10¹² virions/ml.

To store the virus, glycerol was added to the purified virus to a final concentration of 15%, mixed gently but effectively, and stored in aliquots at -80°C.

A protocol developed by Quantum Biotechnologies, Inc. (Montreal, Canada) was followed to measure recombinant virus infectivity. Briefly, two 96-well tissue culture plates were seeded with 1X10⁴ 293A cells per well in MEM containing 2% fetal bovine serum for each recombinant virus to be assayed. After 24 hours 10-fold dilutions of each virus from 1 X 10⁻² to 1 X 10⁻¹⁴ were made in MEM containing 2% fetal bovine serum. 100 µl of each dilution was placed in each of 20 wells. After 5 days at 37°C, wells were read either positive or negative for CPE, and a value for plaque forming units (pfu)/ml was calculated.

TCID₅₀ formulation used was as per Quantum Biotechnologies, Inc., above. The titer (T) was determined from a plate where virus was diluted from 10⁻² to 10⁻¹⁴, and read 5 days after the infection. At each dilution a ratio (R) of positive wells for CPE per the total number of wells was determined. To calculate titer of the undiluted virus sample: the factor, "F' = 1+d(S-0.5); where "S" is the sum of the ratios (R); and "d" is Log10 of the dilution series, for example, "d" is equal to 1 for a ten-fold dilution series. The titer of the undiluted sample is T = 10^{(1+F)} = TCID₅₀/ml. To convert TCID₅₀/ml to pfu/ml, 0.7 is subtracted from the exponent in the calculation for titer (T).

The zvegf3 adenovirus had a titer of 1.8 X 10¹⁰ pfu/ml.

### Example 10

Polyclonal anti-peptide antibodies were prepared by immunizing two female New Zealand white rabbits with the peptides huzvegf3-1 (residues 80-104 of SEQ ID NO:2) huzvegf3-2 (residues 299-314 of SEQ ID NO:2), huzvegf3-3 (residues 299-326 of SEQ ID NO:2 with an N-terminal cys residue), or huzvegf3-4 (residues 195-225 of SEQ ID NO:2 with a C-terminal cys residue). The peptides were synthesized using an Applied Biosystems Model 431A peptide synthesizer (Applied Biosystems, Inc., Foster City, CA) according to the manufacturer's instructions. The peptides huzvegf3-1, huzvegf3-3, and huzvegf3-4 were then conjugated to the carrier protein maleimide-activated keyhole limpet hemocyanin (KLH) through cysteine residues (Pierce Chemical Co., Rockford, IL). The peptide huzvegf3-2 was conjugated to the carrier protein KLH using gluteraldehyde. The rabbits were each given an initial intraperitoneal (IP) injection of 200 µg of conjugated peptide in Complete Freund's Adjuvant (Pierce Chemical Co.) followed by booster IP injections of 100 µg conjugated peptide in Incomplete Freund's Adjuvant every three weeks. Seven to ten days after the administration of the third booster injection, the animals were bled and the serum was collected. The rabbits were then boosted and bled every three weeks.

The huzvegf3 peptide-specific antibodies were affinity purified from the rabbit serum using a CNBr-Sepharose® 4B peptide column (Pharmacia Biotech) that was prepared using 10 mg of the respective peptides per gram CNBr-Sepharose®; followed by dialysis in PBS overnight: Peptide specific-huzvegf3 antibodies were characterized by an ELISA titer check using 1 µg/ml of the appropriate peptide as an antibody target. The huzvegf3-1 peptide-specific antibodies had a lower limit of detection (LLD) of 500 pg/ml by ELISA on the appropriate antibody target and recognized full-length recombinant protein (MBP-fusion) by ELISA. The huzvegf3-2 peptide-specific antibodies had an LLD of 1 ng/ml by ELISA. The huzvegf3-3 peptide-specific antibodies had an LLD of 50 pg/ml by ELISA and recognized recombinant protein by Western Blot analysis. The huzvegf3-4 peptide-specific antibodies had an LLD of 50 pg/ml by ELISA and recognized recombinant protein by Western Blot analysis.

### Example 11

Polyclonal antisera, designated "E2243", was raised in a rabbit by immunization with a full-length human zvegf3 polypeptide fused to *E. coli* maltose binding protein (MBP) and affinity purified using the fusion protein. Specificity of the antisera was examined in a Western blot format in which samples of various zvegf3 proteins were reduced and electrophoresed on a polyacrylamide gel. The proteins used were: recombinant human zvegf3 growth factor domain, recombinant human zvegf3 full-length, and recombinant human zvegf3 full-length fused to MBP, each at concentrations of 13.9, 41.7, and 125 ng/lane; and conditioned media from HaCat cells expressing full-length human zvegf3. The electrophoresed proteins were then transferred to a nitrocellulose membrane, rinsed, and blocked by overnight incubation in buffer containing 2.5% non-fat dry milk. The primary antibody (E2243 antisera) was diluted to 300 ng/ml and added to the nitrocellulose blot, which was then incubated for 1 hour at room temperature with shaking. The blot was then rinsed, secondary antibody (anti-rabbit IgG conjugated to horseradish peroxidase) was added, and the blot was incubated for 1 hour at room temperature with shaking. The blot was then rinsed, developed with commercially available substrates, and exposed to film for 10 seconds. The Western blot showed that the E2243 antisera recognized all samples of full-length zvegf3 (fused and unfused) and the zvegf3 in the conditioned media, but did not recognize any of the samples of isolated zvegf3 growth factor domain.

### Example 12

Mouse hybridomas producing monoclonal antibodies (MAbs) specific for recombinant human zvegf3 growth factor domain (GFD) protein were generated using purified, untagged, recombinant human zvegf3 GFD produced in BHK cells (huzvegf3-GFD-BHK). Ten BALB/c mice were each injected IP on day 1 with 20µg of huzvegf3-GFD-BHK mixed 1:1 (v/v) in complete Freund's adjuvant. Each mouse was subsequently injected IP with 10. µg of huzvegf3-GFD-BHK mixed 1:1 in incomplete Freund's adjuvant on days 15, 29; 41, 57, 71, 89 and 115. On day 118, splenocytes and lymphocytes from enlarged lymph nodes of two mice with the highest anti-huzvegf3 antibody titer (as determined in a biotinylated huzvegf3-GFD capture ELISA; see below) were fused at a 2.76:1 ratio with the X63-Ag8.653 mouse myeloma cell line (Kearney et al., J. Immunol. 123:1548-1550, 1979) essentially as disclosed by Lane (J. Immunol. Methods 81:223-228, 1985). The fusion mixture was plated into 24 96-well plates at an average density of 1.2 x 10⁵ total cells/well in Iscove's modified Dulbecco's medium (IMDM; Life Technologies, Inc., Gaithersburg, MD) containing 10% fetal clone I serum (HyClone Laboratories, Inc., Logan, UT), 10% hybridoma cloning supplement (BM Condimed® H1; Roche Diagnostics Corp., Indianapolis, IN), 2 mM L-glutamine (Life Technologies, Inc.), 100 U/mL penicillin G sodium (Life Technologies, Inc.), and 100 µg/mL streptomycin sulfate (Life Technologies, Inc.). Wells were fed on days 4 and 7 by aspiration and replacement of approximately three-fourths of the media contents in each well. This fusion was designated HH1.

Anti-huzvegf3 mAbs of the IgG class were detected on days 9/10 post-fusion using a biotinylated huzvegf3-GFD capture ELISA. Wells of plates (Immulon® II; Dynex Technologies, Chantilly, VA) were coated with 1 µg/mL of goat anti-mouse IgG (obtained from Kirkegaard & Perry Laboratories, Gaithersburg, MD) in 0.05 M carbonate/bicarbonate buffer (Sigma, St. Louis, MO), 50 µl/well. Plates were incubated at 4°C overnight, then washed three times with phosphate buffered saline containing 0.05% polyoxyethylenesorbitan monolaurate (Tween 20) (PBST) to remove unbound antibody. Wells were blocked with PBST containing 1% (w/v) bovine serum albumin (Sigma) (PTB buffer), 200 µL/well, for 1 hour at room temperature (RT), then washed as above. Culture supernatants from the fusion plates were replica-plated onto antibody-coated plates, 100 uL/well, and incubated at RT for 1 hour. Human zvegf3-GFD protein was biotinylated with sulfo-NHS-LC-biotin (Pierce Chemical Company, Rockford, IL) according to the manufacturer's instructions for 45 minutes at RT. The reaction was stopped with 2M glycine. Biotinylated protein was diluted to 1 µg/mL in PTB buffer. The plates were washed four times with PBST, biotinylated huzvegf3-GFD was added at 100 µL/well, and the plates were incubated at RT for 1 hour. The plates were then washed four times with PBST, and HRP-conjugated streptavidin (Pierce Chemical Company) was diluted to 0.5 µg/mL in PTB buffer and added to the plates at 100 uL/well. The plates were incubated at RT for 1 hour, then washed five times with PBST. TMB substrate (EIA chromagen, Cat. # R6R; Genetic Systems Corp.) was diluted 1:100 in substrate buffer (Genetic Systems Corp.) and added to the plates at 100 µL/well. The plates were incubated 10-15 minutes at RT in the dark. Color development was stopped by the addition of 100 µL/well of 1N H₂SO₄. Optical density was read on an ELISA plate reader (Molecular Devices, Sunnyvale, CA) at wavelengths 450 nm (L1) and 650 nm (L2). Final OD measurement was determined by the formula L1-L2.

78 master wells contained antibody that was capable of capturing biotinylated huzvegf3-GFD. These master wells were expanded for hybridoma cryopreservation and additional supernatant generation. ELISA analysis of the expanded master well supernatants demonstrated that 27 of the original 78 master wells retained antibody specific for huzvegf3-GFD, with 20 of 27 possessing significant reactivity with the antigen.

Six of the designated master wells were cloned by limiting dilution at a density of less than one cell per well. Monoclonality was assessed by microscopic examination of wells for a single focus of cell growth. Clones were tested for specific antibody by the same assay used in the master screen. Five to six of the strongest clones were briefly expanded. Supernatants from these clones were then serially diluted and tested by ELISA to identify the three best antibody-producing clones. Upon verification, the best clone from each master well was subsequently recloned and assayed as described above.

Six of the highest titered second round clones were adapted to growth in fusion/cloning medium minus the addition of hybridoma cloning supplement. Each of the clones was subsequently adapted to growth in a production medium formulation consisting essentially of Dulbecco's modified Eagle's medium + 2.5% fetal clone I serum and various supplements. Supernatants were again serially diluted and tested by ELISA on huzvegf3-GFD to identify the highest titered and next highest titered clones (designated the primary and secondary clones, respectively): MAb produced by each set of clones was then evaluated for IgG subclass using the Mouse Hybridoma Subtyping Kit (Cat. #1183117; Roche Diagnostics Corp.). Clones HH1-24, -40, -57 and 76 were all found to produce an IgG₁ antibody. Clones HH1-58 and -78 produced an IgG_{2b} antibody. All antibodies possessed a κ light chain.

### Example 13

A study was undertaken to test whether adenovirally delivered zvegf3 stimulated cell proliferation as determined by incorporation of bromodeoxyuridine (BrdU) into tissues.

Mice (male, C57B1, 7 weeks old) were divided into three groups. On day 0, parental or zvegf3 adenovirus was administered to the first (n=11) and second (n=12) groups, respectively, via the tail vein, with each mouse receiving a dose of ~1 x 10¹¹ particles in ~0.1 ml volume. The third group (n=8) received no treatment. Each mouse was given two intraperitoneal :doses of 3 mg of freshly made BrdU solution at approximately 24 and 12 hours prior to sacrifice. On days 2, 4, 6, 8, and 10, two mice from each treatment group and one or two untreated mice were sacrificed, and tissues and blood were harvested. Samples were analyzed for complete blood count (CBC) and serum chemistry, and slides were prepared for manual differential blood and marrow progenitor cell analysis. One femur, lung, heart, thymus, liver, kidney, spleen, pancreas, duodenum, and mesenteric lymph nodes were submitted for standard histology and assessment of BrdU incorporation. The lining of the duodenum served as the control tissue for BrdU incorporation.

In addition, two mice that received approximately half the dose of zvegf3 adenovirus particles and one mouse that received the full dose of parental adenovirus were sacrificed and analyzed as described above on day 16.

A piece of liver from each mouse was saved for mRNA assay of adenovirus protein to examine the time course of expression of the adenovirus preparations.

Beginning on day 6, most of the animals treated with either adenovirus had visibly enlarged livers and spleens compared to the untreated mice. The livers of the zvegf3 adenovirus-treated mice tended to look more pale than animals treated with the parental virus. Proliferation of sinusoidal cells was observed in liver. Visual inspection suggested that these cells were stellate cells and/or fibroblasts. Spleen color was the same in both groups. Most of the animals that received the zvegf3 adenovirus had paler femur shafts, with the marrow lighter in color.

Peripheral blood CBCs showed a possible difference in platelet counts, but not in RBC or WBC counts between zvegf3 and parental virus-treated animals. In comparison to the untreated and parental virus-treated groups, the zvegf3 group had lower platelet counts on days 2, 4, 6, and 8, but not on day 10. The mean platelet volume (average size of individual platelets) in the zvegf3 group also tended to be greater, consistent with a relative increase in the larger, immature platelet population.

BrdU labeling showed increased cell proliferation in kidney, mainly in the medulla and to a lesser extent in the cortex. Proliferating cells appeared to be interstitial cells, which may have included fibroblasts and/or mesangial cells.

### Example 14

Human zvegf3 growth factor domain protein produced in BHK cells was tested for the ability to stimulate production of TGF-β in stellate cells. Rat hepatic stellate cells (obtained from Dr. Nelson Fausto, University of Washington) were plated in 48-well tissue culture clusters (Costar®; Corning, Corning, NY) in DMEM growth medium. (Life Technologies, Inc.) supplemented with pyruvate and 10% serum (HyClone Laboratories, Inc.). At confluence, the medium was changed to serum-free medium by substituting 0.1% BSA (Fraction V; Sigma, St. Louis, MO) for serum. 48 hours later, the medium was changed, replaced with the same serum-free medium, and the zvegf3 GFD protein was added at 100 ng/ml in the wells. 48 hours later, conditioned media were collected and spun down to get rid of any floating cells or debris. Total TGF-β1 levels were determined in these media using a commercially available ELISA kit (R&D Systems, St Paul, MN). Stimulation of stellate cells with 100 ng/ml zvegf3 GFD resulted in an approximately 5-fold increase in the production of TGF-β compared to a BSA control.

## Claims

1. The use of a zvegf3 antagonist in the manufacture of a medicament for treating hepatocellular carcinoma in a mammal, e.g., wherein tumor response is to be measured as a complete response, a partial response, or a reduction in time to progression, and wherein the antagonist is an anti-zvegf3 antibody which specifically binds to a dimeric protein having two polypeptide chains, wherein each of said polypeptide chains consist of a sequence of amino acid residues selected from the group consisting of:
residues 230-345 of SEQ ID NO:2;
residues 231-345 of SEQ ID NO:2;
residues 232-345 of SEQ ID NO:2;
residues 233-345 of SEQ ID NO:2;
residues 234-345 of SEQ ID NO:2;
residues 235-345 of SEQ ID NO:2;
residues 236-345 of SEQ ID NO:2;
residues 237-345 of SEQ ID NO:2;
residues 238-345 of SEQ ID NO:2;
residues 239-345 of SEQ ID NO:2; and
residues 240-345 of SEQ ID NO:2.

2. The use of a zvegf3 antagonist in the manufacture of a medicament for reducing cancer cell proliferation in a mammal with hepatocellular carcinoma, wherein the antagonist is an anti-zvegf3 antibody which specifically binds to a dimeric protein having two polypeptide chains, wherein each of said polypeptide chains consist of a sequence of amino acid residues selected from the group consisting of:
residues 230-345 of SEQ ID NO:2;
residues 231-345 of SEQ ID NO:2;
residues 232-345 of SEQ ID NO:2;
residues 233-345 of SEQ ID NO:2;
residues 234-345 of SEQ ID NO:2;
residues 235-345 of SEQ ID NO:2;
residues 236-345 of SEQ ID NO:2;
residues 237-345 of SEQ ID NO:2;
residues 238-345 of SEQ ID NO:2;
residues 239-345 of SEQ ID NO:2; and
residues 240-345 of SEQ ID NO:2.

3. The use of claim 1 or 2 wherein the anti-zvegf3 antibody is a monoclonal antibody.

4. The use of claim 3 wherein the monoclonal antibody is an IgG antibody.

5. The use of any one of claims 1 to 4 wherein the medicament is for administration of the zvegf3 antagonist by, intravenous infusion.

6. The zvegf3 antagonist as defined by claim 1 for use in treating hepatocellular carcinoma in a mammal or reducing cancer cell proliferation in a mammal with hepatocellular carcinoma, optionally wherein the antibody is further defined by the features of any of claims 3 to 5.

## Patentansprüche

1. Die Verwendung eines zvegf3-Antagonisten bei der Herstellung eines Medikaments zur Behandlung eines hepatozellulären Karzinoms in einem Säuger, wobei z.B. die Tumorantwort als eine vollständige Antwort, eine partielle Antwort oder eine Verringerung bei der Zeit bis zur Progression gemessen werden soll und wobei der Antagonist ein anti-zvegf3-Antikörper ist, welcher spezifisch an ein dimeres Protein, das zwei Polypeptidketten aufweist, bindet, wobei jede der Polypeptidketten aus einer Sequenz von Aminosäurenresten besteht, die ausgewählt ist aus der Gruppe, bestehend aus:
den Resten 230-345 der SEQ ID NO:2;
den Resten 231-345 der SEQ ID NO:2;
den Resten 232-345 der SEQ ID NO:2;
den Resten 233-345 der SEQ ID NO:2;
den Resten 234-345 der SEQ ID NO:2;
den Resten 235-345 der SEQ ID NO:2;
den Resten 236-345 der SEQ ID NO:2;
den Resten 237-345 der SEQ ID NO:2;
den Resten 238-345 der SEQ ID NO:2;
den Resten 239-345 der SEQ ID NO:2; und
den Resten 240-345 der SEQ ID NO:2.

2. Die Verwendung eines zvegf3-Antagonisten bei der Herstellung eines Medikaments zur Verringerung der Krebszellenproliferation in einem Säuger mit hepatozellulärem Karzinom, wobei der Antagonist ein anti-zvegf3-Antikörper ist, welcher spezifisch an ein dimeres Protein, das zwei Polypeptidketten aufweist, bindet, wobei jede der Polypeptidketten aus einer Sequenz von Aminosäurenresten besteht, die ausgewählt ist aus der Gruppe, bestehend aus:
den Resten 230-345 der SEQ ID NO:2;
den Resten 231-345 der SEQ ID NO:2;
den Resten 232-345 der SEQ ID NO:2;
den Resten 233-345 der SEQ ID NO:2;
den Resten 234-345 der SEQ ID NO:2;
den Resten 235-345 der SEQ ID NO:2;
den Resten 236-345 der SEQ ID NO:2;
den Resten 237-345 der SEQ ID NO:2;
den Resten 238-345 der SEQ ID NO:2;
den Resten 239-345 der SEQ ID NO:2; und
den Resten 240-345 der SEQ ID NO:2.

3. Die Verwendung nach Anspruch 1 oder 2, wobei der anti-zvegf3-Antikörper ein monoklonaler Antikörper ist.

4. Die Verwendung nach Anspruch 3, wobei der monoklonale Antikörper ein IgG-Antikörper ist.

5. Die Verwendung nach irgendeinem der Ansprüche 1 bis 4, wobei das Medikament zur Verabreichung des zvegf3-Antagonisten durch intravenöse Infusion dient.

6. Der zvegf3-Antagonist wie in Anspruch 1 definiert zur Verwendung bei der Behandlung eines hepatozellulären Karzinoms in einem Säuger oder der Verringerung der Krebszellenproliferation in einem Säuger mit hepatozellulärem Karzinom, wobei der Antikörper gegebenenfalls weiter durch die Merkmale von irgendeinem der Ansprüche 3 bis 5 definiert ist.

## Revendications

1. Utilisation d'un antagoniste de zvegf3 dans la fabrication d'un médicament pour le traitement du carcinome hépatocellulaire chez un mammifère, par exemple, où la réponse tumorale doit être mesurée comme une réponse complète, une réponse partielle, ou une réduction dans le temps de la progression, et où l'antagoniste est un anticorps anti-zvegf3 qui se lie spécifiquement à une protéine dimérique ayant deux chaînes polypeptidiques, où chacune desdites chaînes polypeptidiques consiste en une séquence de résidus acides aminés sélectionnés parmi le groupe consistant en :
résidus 230-345 de la SEQ ID N° : 2 ;
résidus 231-345 de la SEQ ID N° : 2 ;
résidus 232-345 de la SEQ ID N° : 2 ;
résidus 233-345 de la SEQ ID N° : 2 ;
résidus 234-345 de la SEQ ID N° : 2 ;
résidus 235-345 de la SEQ ID N° : 2 ;
résidus 236-345 de la SEQ ID N° : 2 ;
résidus 237-345 de la SEQ ID N° : 2 ;
résidus 238-345 de la SEQ ID N° : 2 ;
résidus 239-345 de la SEQ ID N° : 2 ; et
résidus 240-345 de la SEQ ID N° : 2 ;

2. Utilisation d'un antagoniste de zvegf3 dans la fabrication d'un médicament pour la réduction de la prolifération des cellules cancéreuses chez un mammifère avec carcinome hépatocellulaire, où l'antagoniste est un anticorps anti-zvegf3 qui se lie spécifiquement à une protéine dimérique ayant deux chaînes polypeptidiques, où chacune desdites chaînes polypeptidiques consiste en une séquence de résidus acides aminés sélectionnés parmi le groupe consistant en :
résidus 230-345 de la SEQ ID N° : 2 ;
résidus 231-345 de la SEQ ID N° : 2 ;
résidus 232-345 de la SEQ ID N° : 2 ;
résidus 233-345 de la SEQ ID N° : 2 ;
résidus 234-345 de la SEQ ID N° : 2 ;
résidus 235-345 de la SEQ ID N° : 2 ;
résidus 236-345 de la SEQ ID N° : 2 ;
résidus 237-345 de la SEQ ID N° : 2 ;
résidus 23 8-345 de la SEQ ID N° : 2 ;
résidus 239-345 de la SEQ ID N° : 2 ; et
résidus 240-345 de la SEQ ID N° : 2 ;

3. Utilisation selon la revendication 1 ou 2, dans laquelle l'anticorps anti-zvegf3 est un anticorps monoclonal.

4. Utilisation selon la revendication 3, dans laquelle l'anticorps monoclonal est un anticorps IgG.

5. Utilisation selon l'une quelconque des revendications 1 à 4, dans laquelle le médicament est destiné à l'administration de l'antagoniste de zvegf3 par perfusion intraveineuse.

6. Antagoniste de zvegf3 selon la revendication 1, destiné à une utilisation dans le traitement du carcinome hépatocellulaire chez un mammifère ou dans la réduction de la prolifération des cellules cancéreuses chez un mammifère avec carcinome hépatocellulaire, éventuellement où l'anticorps est en outre défini par les caractéristiques selon l'une quelconque des revendications 3 à 5.


### SEQUENCE LISTING

<110> ZymoGenetics, Inc.
<120> METHOD OF TREATING HEPATOCELLULAR CARCINOMA
<130> 03-14PC
<150> 60/490,047
   <151> 2003-07-25
<160> 18
<170> FastSEQ for Windows Version 3.0
<210> 1
   <211> 1760
   <212> DNA
   <213> Homo sapiens
<220>
   <221> CDS
   <222> (154)...(1191)
<400> 1
   attatgtgga aactaccctg cgattctctg ctgccagagc aggctcggcg cttccacccc      60
   agtgcagcct tcccctggcg gtggtgaaag agactcggga gtcgctgctt ccaaagtgcc     120
   cgccgtgagt gagctctcac cccagtcagc caa atg agc ctc ttc ggg ctt ctc      174
                                        Met Ser Leu Phe Gly Leu Leu
                                        1               5
   ctg ctg aca tct gcc ctg gcc ggc cag aga cag ggg act cag gcg gaa      222
   Leu Leu Thr Ser Ala Leu Ala Gly Gln Arg Gln Gly Thr Gln Ala Glu
           10                  15                  20
   tcc aac ctg agt agt aaa ttc cag ttt tcc agc aac aag gaa cag aac      270
   Ser Asn Leu Ser Ser Lys Phe Gln Phe Ser Ser Asn Lys Glu Gln Asn
       25                  30                  35
   gga gta caa gat cct cag cat gag aga att att act gtg tct act aat      318
   Gly Val Gln Asp Pro Gln His Glu Arg Ile Ile Thr Val Ser Thr Asn
   40                  45                  50                  55
   gga agt att cac agc cca agg ttt cct cat act tat cca aga aat acg      366
   Gly Ser Ile His Ser Pro Arg Phe Pro His Thr Tyr Pro Arg Asn Thr
                    60                  65                  70
   gtc ttg gta tgg aga tta gta gca gta gag gaa aat gta tgg ata caa      414
   Val Leu Val Trp Arg Leu Val Ala Val Glu Glu Asn Val Trp Ile Gln
                75                  80                  85
   ctt acg ttt gat gaa aga ttt ggg ctt gaa gac cca gaa gat gac ata      462
   Leu Thr Phe Asp Glu Arg Phe Gly Leu Glu Asp Pro Glu Asp Asp Ile
            90                  95                 100
   tgc aag tat gat ttt gta gaa gtt gag gaa ccc agt gat gga act ata      510
   Cys Lys Tyr Asp Phe Val Glu Val Glu Glu Pro Ser Asp Gly Thr Ile
       105                 110                 115
   tta ggg cgc tgg tgt ggt tct ggt act gta cca gga aaa cag att tct      558
   Leu Gly Arg Trp Cys Gly Ser Gly Thr Val Pro Gly Lys Gln Ile Ser
   120                 125                 130                 135
   aaa gga aat caa att agg ata aga ttt gta tct gat gaa tat ttt cct      606
   Lys Gly Asn Gln Ile Arg Ile Arg Phe Val Ser Asp Glu Tyr Phe Pro
                   140                 145                 150
   tct gaa cca ggg ttc tgc atc cac tac aac att gtc atg cca caa ttc      654
   Ser Glu Pro Gly Phe Cys Ile His Tyr Asn Ile Val Met Pro Gln Phe
               155                 160                 165
   aca gaa gct gtg agt cct tca gtg cta ccc cct tca gct ttg cca ctg      702
   Thr Glu Ala Val Ser Pro Ser Val Leu Pro Pro Ser Ala Leu Pro Leu
           170                 175                 180
   gac ctg ctt aat aat gct ata act gcc ttt agt acc ttg gaa gac ctt      750
   Asp Leu Leu Asn Asn Ala Ile Thr Ala Phe Ser Thr Leu Glu Asp Leu
       185                 190                 195
   att cga tat ctt gaa cca gag aga tgg cag ttg gac tta gaa gat cta      798
   Ile Arg Tyr Leu Glu Pro Glu Arg Trp Gln Leu Asp Leu Glu Asp Leu
   200                 205                 210                 215
   tat agg cca act tgg caa ctt ctt ggc aag gct ttt gtt ttt gga aga      846
   Tyr Arg Pro Thr Trp Gln Leu Leu Gly Lys Ala Phe Val Phe Gly Arg
                   220                 225                 230
   aaa tcc aga gtg gtg gat ctg aac ctt cta aca gag gag gta aga tta       894
   Lys Ser Arg Val Val Asp Leu Asn Leu Leu Thr Glu Glu Val Arg Leu
               235                 240                 245
   tac agc tgc aca cct cgt aac ttc tca gtg tcc ata agg gaa gaa cta       942
   Tyr Ser Cys Thr Pro Arg Asn Phe Ser Val Ser Ile Arg Glu Glu Leu
           250                 255                 260
   aag aga acc gat acc att ttc tgg cca ggt tgt ctc ctg gtt aaa cgc       990
   Lys Arg Thr Asp Thr Ile Phe Trp Pro Gly Cys Leu Leu Val Lys Arg
       265                 270                 275
   tgt ggt ggg aac tgt gcc tgt tgt ctc cac aat tgc aat gaa tgt caa      1038
   Cys Gly Gly Asn Cys Ala Cys Cys Leu His Asn Cys Asn Glu Cys Gln
   280                 285                 290                 295
   tgt gtc cca agc aaa gtt act aaa aaa tac cac gag gtc ctt cag ttg      1086
   Cys Val Pro Ser Lys Val Thr Lys Lys Tyr His Glu Val Leu Gln Leu
                   300                 305                 310
   aga cca aag acc ggt gtc agg gga ttg cac aaa tca ctc acc gac gtg      1134
   Arg Pro Lys Thr Gly Val Arg Gly Leu His Lys Ser Leu Thr Asp Val
               315                 320                 325
   gcc ctg gag cac cat gag gag tgt gac tgt gtg tgc aga ggg agc aca      1182
   Ala Leu Glu His His Glu Glu Cys Asp Cys Val Cys Arg Gly Ser Thr
           330                 335                 340
   gga gga tag ccgcatcacc accagcagct cttgcccaga gctgtgcagt              1231
   Gly Gly  *
       345
   gcagtggctg attctattag agaacgtatg cgttatctcc atccttaatc tcagttgttt    1291
   gcttcaagga cctttcatct tcaggattta cagtgcattc tgaaagagga gacatcaaac    1351
   agaattagga gttgtgcaac agctcttttg agaggaggcc taaaggacag gagaaaaggt    1411
   cttcaatcgt ggaaagaaaa ttaaatgttg tattaaatag atcaccagct agtttcagag    1471
   ttaccatgta cgtattccac tagctgggtt ctgtatttca gttctttcga tacggcttag    1531
   ggtaatgtca gtacaggaaa aaaactgtgc aagtgagcac ctgattccgt tgccttgctt    1591
   aactctaaag ctccatgtcc tgggcctaaa atcgtataaa atctggattt tttttttttt    1651
   tttttgctca tattcacata tgtaaaccag aacattctat gtactacaaa cctggttttt    1711
   aaaaaggaac tatgttgcta tgaattaaac ttgtgtcgtg ctgatagga                1760
<210> 2
   <211> 345
   <212> PRT
   <213> Homo sapiens
<400> 2
   Met Ser Leu Phe Gly Leu Leu Leu Leu Thr Ser Ala Leu Ala Gly Gln
   1               5                   10                  15
   Arg Gln Gly Thr Gln Ala Glu Ser Asn Leu Ser Ser Lys Phe Gln Phe
               20                  25                  30
   Ser Ser Asn Lys Glu Gln Asn Gly Val Gln Asp Pro Gln His Glu Arg
           35                  40                  45
   Ile Ile Thr Val Ser Thr Asn Gly Ser Ile His Ser Pro Arg Phe Pro
       50                  55                  60
   His Thr Tyr Pro Arg Asn Thr Val Leu Val Trp Arg Leu Val Ala Val
   65                  70                  75                  80
   Glu Glu Asn Val Trp Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Thr Ile Leu Gly Arg Trp Cys Gly Ser Gly Thr
           115                 120                 125
   Val Pro Gly Lys Gln Ile Ser Lys Gly Asn Gln Ile Arg Ile Arg Phe
       130                 135                 140
   Val Ser Asp Glu Tyr Phe Pro Ser Glu Pro Gly Phe Cys Ile His Tyr
   145                 150                 155                 160
   Asn Ile Val Met Pro Gln Phe Thr Glu Ala Val Ser Pro Ser Val Leu
                   165                 170                 175
   Pro Pro Ser Ala Leu Pro Leu Asp Leu Leu Asn Asn Ala Ile Thr Ala
               180                 185                 190
   Phe Ser Thr Leu Glu Asp Leu Ile Arg Tyr Leu Glu Pro Glu Arg Trp
           195                 200                 205
   Gln Leu Asp Leu Glu Asp Leu Tyr Arg Pro Thr Trp Gln Leu Leu Gly
       210                 215                 220
   Lys Ala Phe Val Phe Gly Arg Lys Ser Arg Val Val Asp Leu Asn Leu
   225                 230                 235                 240
   Leu Thr Glu Glu Val Arg Leu Tyr Ser Cys Thr Pro Arg Asn Phe Ser
                   245                 250                 255
   Val Ser Ile Arg Glu Glu Leu Lys Arg Thr Asp Thr Ile Phe Trp Pro
               260                 265                 270
   Gly Cys Leu Leu Val Lys Arg Cys Gly Gly Asn Cys Ala Cys Cys Leu
           275                 280                 285
   His Asn Cys Asn Glu Cys Gln Cys Val Pro Ser Lys Val Thr Lys Lys
       290                 295                 300
   Tyr His Glu Val Leu Gln Leu Arg Pro Lys Thr Gly Val Arg Gly Leu
   305                 310                 315                 320
   His Lys Ser Leu Thr Asp Val Ala Leu Glu His His Glu Glu Cys Asp
                   325                 330                 335
   Cys Val Cys Arg Gly Ser Thr Gly Gly
               340                 345
<210> 3
   <211> 3571
   <212> DNA
   <213> Mus musculus
<220>
   <221> CDS
   <222> (1049)...(2086)
<400> 3
   gaattcccgg gtcgacccac gcgtccgggc gcccagggga aaggaagctg ggggccgcct      60
   ggcggcattc ctcgccgcag tgtgggctcc gtctgccgcg gggcccgcag tgccccctgt     120
   ctgcgccagc acctgttggc ccgccagctg gccgcccgcg ccccccgcgc cccccgcgcc     180
   cgcccggccg ccagccccgc gccccgcgcg ccgcccgctg ggggaaagtg gagacgggga     240
   ggggacaaga gcgatcctcc aggccagcca ggccttccct tagccgcccg tgcttagccg     300
   ccacctctcc tcagccctgc gtcctgccct gccttagggc aggcatccga gcgctcgcga     360
   ctccgagccg cccaagctct cccggcttcc cgcagcactt cgccggtacc cgagggaact     420
   tcggtggcca ccgactgcag caaggaggag gctccgcggt ggatccgggc cagtcccgag     480
   tcgtccccgc ggcctctctg cccgcccggg acccgcgcgg cactcgcagg gcacggtccc     540
   ctccccccag gtgggggtgg ggcgccgcct gccgccccga tcagcagctt tgtcattgat     600
   cccaaggtgc tcgcctcgct gccgacctgg cttccagtct ggcttggcgg gaccccgagt     660
   cctcgcctgt gtcctgtccc ccaaactgac aggtgctccc tgcgagtcgc cacgactcat     720
   cgccgctccc ccgcgtcccc accccttctt tcctccctcg cctaccccca ccccccgcac     780
   ttcggcacag ctcaggattt gtttaaacct tgggaaactg gttcaggtcc aggttttgct     840
   ttgatccttt tcaaaaactg gagacacaga agagggctct aggaaaaact tttggatggg     900
   attatgtgga aactaccctg cgattctctg ctgccagagc cggccaggcg cttccaccgc     960
   agcgcagcct ttccccggct gggctgagcc ttggagtcgt cgcttcccca gtgcccgccg    1020
   cgagtgagcc ctcgccccag tcagccaa atg ctc ctc ctc ggc ctc ctc ctg      1072
                                  Met Leu Leu Leu Gly Leu Leu Leu
                                  1               5
   ctg aca tct gcc ctg gcc ggc caa aga acg ggg act cgg gct gag tcc     1120
   Leu Thr Ser Ala Leu Ala Gly Gln Arg Thr Gly Thr Arg Ala Glu Ser
       10                  15                  20
   aac ctg agc agc aag ttg cag ctc tcc agc gac aag gaa cag aac gga     1168
   Asn Leu Ser Ser Lys Leu Gln Leu Ser Ser Asp Lys Glu Gln Asn Gly
   25                  30                  35                  40
   gtg caa gat ccc cgg cat gag aga gtt gtc act ata tct ggt aat ggg      1216
   Val Gln Asp Pro Arg His Glu Arg Val Val Thr Ile Ser Gly Asn Gly
                   45                  50                  55
   agc atc cac agc ccg aag ttt cct cat aca tac cca aga aat atg gtg      1264
   Ser Ile His Ser Pro Lys Phe Pro His Thr Tyr Pro Arg Asn Met Val
               60                  65                  70
   ctg gtg tgg aga tta gtt gca gta gat gaa aat gtg cgg atc cag ctg      1312
   Leu Val Trp Arg Leu Val Ala Val Asp Glu Asn Val Arg Ile Gln Leu
           75                  80                  85
   aca ttt gat gag aga ttt ggg ctg gaa gat cca gaa gac gat ata tgc      1360
   Thr Phe Asp Glu Arg Phe Gly Leu Glu Asp Pro Glu Asp Asp Ile Cys
       90                  95                  100
   aag tat gat ttt gta gaa gtt gag gag ccc agt gat gga agt gtt tta      1408
   Lys Tyr Asp Phe Val Glu Val Glu Glu Pro Ser Asp Gly Ser Val Leu
   105                 110                 115                 120
   gga cgc tgg tgt ggt tct ggg act gtg cca gga aag cag act tct aaa      1456
   Gly Arg Trp Cys Gly Ser Gly Thr Val Pro Gly Lys Gln Thr Ser Lys
                   125                 130                 135
   gga aat cat atc agg ata aga ttt gta tct gat gag tat ttt cca tct      1504
   Gly Asn His Ile Arg Ile Arg Phe Val Ser Asp Glu Tyr Phe Pro Ser
               140                 145                 150
   gaa ccc gga ttc tgc atc cac tac agt att atc atg cca caa gtc aca      1552
   Glu Pro Gly Phe Cys Ile His Tyr Ser Ile Ile Met Pro Gln Val Thr
           155                 160                 165
   gaa acc acg agt cct tcg gtg ttg ccc cct tca tct ttg tca ttg gac      1600
   Glu Thr Thr Ser Pro Ser Val Leu Pro Pro Ser Ser Leu Ser Leu Asp
       170                 175                 180
   ctg ctc aac aat gct gtg act gcc ttc agt acc ttg gaa gag ctg att      1648
   Leu Leu Asn Asn Ala Val Thr Ala Phe Ser Thr Leu Glu Glu Leu Ile
   185                 190                 195                 200
   cgg tac cta gag cca gat cga tgg cag gtg gac ttg gac agc ctc tac      1696
   Arg Tyr Leu Glu Pro Asp Arg Trp Gln Val Asp Leu Asp Ser Leu Tyr
                   205                 210                 215
   aag cca aca tgg cag ctt ttg ggc aag gct ttc ctg tat ggg aaa aaa       1744
   Lys Pro Thr Trp Gln Leu Leu Gly Lys Ala Phe Leu Tyr Gly Lys Lys
               220                 225                 230
   agc aaa gtg gtg aat ctg aat ctc ctc aag gaa gag gta aaa ctc tac       1792
   Ser Lys Val Val Asn Leu Asn Leu Leu Lys Glu Glu Val Lys Leu Tyr
           235                 240                 245
   agc tgc aca ccc cgg aac ttc tca gtg tcc ata cgg gaa gag cta aag       1840
   Ser Cys Thr Pro Arg Asn Phe Ser Val Ser Ile Arg Glu Glu Leu Lys
       250                 255                 260
   agg aca gat acc ata ttc tgg cca ggt tgt ctc ctg gtc aag cgc tgt       1888
   Arg Thr Asp Thr Ile Phe Trp Pro Gly Cys Leu Leu Val Lys Arg Cys
   265                 270                 275                 280
   gga gga aat tgt gcc tgt tgt ctc cat aat tgc aat gaa tgt cag tgt       1936
   Gly Gly Asn Cys Ala Cys Cys Leu His Asn Cys Asn Glu Cys Gln Cys
                   285                 290                 295
   gtc cca cgt aaa gtt aca aaa aag tac cat gag gtc ctt cag ttg aga       1984
   Val Pro Arg Lys Val Thr Lys Lys Tyr His Glu Val Leu Gln Leu Arg
               300                 305                 310
   cca aaa act gga gtc aag gga ttg cat aag tca ctc act gat gtg gct       2032
   Pro Lys Thr Gly Val Lys Gly Leu His Lys Ser Leu Thr Asp Val Ala
           315                 320                 325
   ctg gaa cac cac gag gaa tgt gac tgt gtg tgt aga gga aac gca gga       2080
   Leu Glu His His Glu Glu Cys Asp Cys Val Cys Arg Gly Asn Ala Gly
       330                 335                 340
   ggg taa ctgcagcctt cgtagcagca cacgtgagca ctggcattct gtgtaccccc       2136
   Gly  *
   345
   acaagcaacc ttcatcccca ccagcgttgg ccgcagggct ctcagctgct gatgctggct     2196
   atggtaaaga tcttactcgt ctccaaccaa attctcagtt gtttgcttca atagccttcc     2256
   cctgcaggac ttcaagtgtc ttctaaaaga ccagaggcac caagaggagt caatcacaaa     2316
   gcactgcctt ctagaggaag cccagacaat ggtcttctga ccacagaaac aaatgaaatg     2376
   aatgtagatc gctagcaaac tctggagtga cagcatttct tttccactga cagaatggtg     2436
   tagcttagtt gtcttgatat gggcaagtga tgtcagcaca agaaaatggt gaaaaacaca     2496
   cacttgattg tgaacaatgc agaaatactt ggatttctcc aacctgtttg catagataga     2556
   cagatgctct gttttctaca aactcaaagc ttttagagag cagctatgtt aataggaatt     2616
   aaatgtgcca tgctgaaagg aaagactgaa gttttcaatg cttggcaact tctccgcaat   2676
   ttggaggaaa ggtgcggtca tggtttggag aaagcacacc tgcacagagg agtggccttc   2736
   ccttcccttc cctctgaggt ggcttctgtg tttcattgtg tatattttta tattctcctt   2796
   ttgacattat aactgttggc ttttctaatc ttgttaaata tttctatttt taccaaaggt   2856
   atttaatatt cttttttatg acaacctaga gcaattattt ttagcttgat aatttttttt    2916
   tctaaacaaa attgttatag ccagaagaac aaagatgatt gatataaaaa tcttgttgct   2976
   ctgacaaaaa catatgtatt tcttccttgt atggtgctag agcttagcgt catctgcatt   3036
   tgaaaagatg gaatggggaa gtttttagaa ttggtaggtc gcagggacag tttgataaca   3096
   actgtactat catcaattcc caattctgtt cttagagcta cgaacagaac agagcttgag   3156
   taaatatgga gccattgcta acctacccct ttctatggga aataggagta tagctcagag   3216
   aagcacgtcc ccagaaacct cgaccatttc taggcacagt gttctgggct atgctgcgct   3276
   gtatggacat atcctattta tttcaatact agggttttat tacctttaaa ctctgctcca   3336
   tacacttgta ttaatacatg gatattttta tgtacagaag tatatcattt aaggagttca   3396
   cttattatac tctttggcaa ttgcaaagaa aatcaacata atacattgct tgtaaatgct   3456
   taatctgtgc ccaagttttg tggtgactat ttgaattaaa atgtattgaa tcatcaaata   3516
   aaataatctg gctattttgg ggaaaaaaaa aaaaaaaaaa aaaaagggcg gccgc        3571
<210> 4
   <211> 345
   <212> PRT
   <213> Mus musculus
<400> 4
   Met Leu Leu Leu Gly Leu Leu Leu Leu Thr Ser Ala Leu Ala Gly Gln
    1               5                  10                  15
   Arg Thr Gly Thr Arg Ala Glu Ser Asn Leu Ser Ser Lys Leu Gln Leu
               20                  25                  30
   Ser Ser Asp Lys Glu Gln Asn Gly Val Gln Asp Pro Arg His Glu Arg
           35                  40                  45
   Val Val Thr Ile Ser Gly Asn Gly Ser Ile His Ser Pro Lys Phe Pro
       50                  55                  60
   His Thr Tyr Pro Arg Asn Met Val Leu Val Trp Arg Leu Val Ala Val
   65                  70                  75                  80
   Asp Glu Asn Val Arg Ile Gln Leu Thr Phe Asp Glu Arg Phe Gly Leu
                   85                  90                  95
   Glu Asp Pro Glu Asp Asp Ile Cys Lys Tyr Asp Phe Val Glu Val Glu
               100                 105                 110
   Glu Pro Ser Asp Gly Ser Val Leu Gly Arg Trp Cys Gly Ser Gly Thr
           115                 120                 125
   Val Pro Gly Lys Gln Thr Ser Lys Gly Asn His Ile Arg Ile Arg Phe
       130                 135                 140
   Val Ser Asp Glu Tyr Phe Pro Ser Glu Pro Gly Phe Cys Ile His Tyr
   145                 150                 155                 160
   Ser Ile Ile Met Pro Gln Val Thr Glu Thr Thr Ser Pro Ser Val Leu
   165 170 175
   Pro Pro Ser Ser Leu Ser Leu Asp Leu Leu Asn Asn Ala Val Thr Ala
   180 185 190
   Phe Ser Thr Leu Glu Glu Leu Ile Arg Tyr Leu Glu Pro Asp Arg Trp
   195 200 205
   Gln Val Asp Leu Asp Ser Leu Tyr Lys Pro Thr Trp Gln Leu Leu Gly
   210 215 220
   Lys Ala Phe Leu Tyr Gly Lys Lys Ser Lys Val Val Asn Leu Asn Leu
   225 230 235 240
   Leu Lys Glu Glu Val Lys Leu Tyr Ser Cys Thr Pro Arg Asn Phe Ser
   245 250 255
   Val Ser Ile Arg Glu Glu Leu Lys Arg Thr Asp Thr Ile Phe Trp Pro
   260 265 270
   Gly Cys Leu Leu Val Lys Arg Cys Gly Gly Asn Cys Ala Cys Cys Leu
   275 280 285
   His Asn Cys Asn Glu Cys Gln Cys Val Pro Arg Lys Val Thr Lys Lys
   290 295 300
   Tyr His Glu Val Leu Gln Leu Arg Pro Lys Thr Gly Val Lys Gly Leu
   305 310 315 320
   His Lys Ser Leu Thr Asp Val Ala Leu Glu His His Glu Glu Cys Asp
   325 330 335
   Cys Val Cys Arg Gly Asn Ala Gly Gly
   340 345
<210> 5
   <211> 370
   <212> PRT
   <213> Homo sapiens
<400> 5
   Met His Arg Leu Ile Phe Val Tyr Thr Leu Ile Cys Ala Asn Phe Cys
   1 5 10 15
   Ser Cys Arg Asp Thr Ser Ala Thr Pro Gln Ser Ala Ser Ile Lys Ala
   20 25 30
   Leu Arg Asn Ala Asn Leu Arg Arg Asp Glu Ser Asn His Leu Thr Asp
   35 40 45
   Leu Tyr Arg Arg Asp Glu Thr Ile Gln Val Lys Gly Asn Gly Tyr Val
   50 55 60
   Gln Ser Pro Arg Phe Pro Asn Ser Tyr Pro Arg Asn Leu Leu Leu Thr
   65 70 75 80
   Trp Arg Leu His Ser Gln Glu Asn Thr Arg Ile Gln Leu Val Phe Asp
   85 90 95
   Asn Gln Phe Gly Leu Glu Glu Ala Glu Asn Asp Ile Cys Arg Tyr Asp
   100 105 110
   Phe Val Glu Val Glu Asp Ile Ser Glu Thr Ser Thr Ile Ile Arg Gly
   115 120 125
   Arg Trp Cys Gly His Lys Glu Val Pro Pro Arg Ile Lys Ser Arg Thr
   130 135 140
   Asn Gln Ile Lys Ile Thr Phe Lys Ser Asp Asp Tyr Phe Val Ala Lys
   145 150 155 160
   Pro Gly Phe Lys Ile Tyr Tyr Ser Leu Leu Glu Asp Phe Gln Pro Ala
   165 170 175
   Ala Ala Ser Glu Thr Asn Trp Glu Ser Val Thr Ser Ser Ile Ser Gly
   180 185 190
   Val Ser Tyr Asn Ser Pro Ser Val Thr Asp Pro Thr Leu Ile Ala Asp
   195 200 205
   Ala Leu Asp Lys Lys Ile Ala Glu Phe Asp Thr Val Glu Asp Leu Leu
   210 215 220
   Lys Tyr Phe Asn Pro Glu Ser Trp Gln Glu Asp Leu Glu Asn Met Tyr
   225 230 235 240
   Leu Asp Thr Pro Arg Tyr Arg Gly Arg Ser Tyr His Asp Arg Lys Ser
   245 250 255
   Lys Val Asp Leu Asp Arg Leu Asn Asp Asp Ala Lys Arg Tyr Ser Cys
   260 265 270
   Thr Pro Arg Asn Tyr Ser Val Asn Ile Arg Glu Glu Leu Lys Leu Ala
   275 280 285
   Asn Val Val Phe Phe Pro Arg Cys Leu Leu Val Gln Arg Cys Gly Gly
   290 295 300
   Asn Cys Gly Cys Gly Thr Val Asn Trp Arg Ser Cys Thr Cys Asn Ser
   305 310 315 320
   Gly Lys Thr Val Lys Lys Tyr His Glu Val Leu Gln Phe Glu Pro Gly
   325 330 335
   His Ile Lys Arg Arg Gly Arg Ala Lys Thr Met Ala Leu Val Asp Ile
   340 345 350
   Gln Leu Asp His His Glu Arg Cys Asp Cys Ile Cys Ser Ser Arg Pro
   355 360 365
   Pro Arg
   370
<210> 6
   <211> 20
   <212> DNA
   <213> Artificial Sequence
<220>
   <223> oligonucleotide primer
<400> 6
   agcaggtcca gtggcaaagc 20
<210> 7
   <211> 21
   <212> DNA
   <213> Artificial Sequence
<220>
   <223> oligonucleotide primer
<400> 7
   cgtttgatga aagatttggg c 21
<210> 8
   <211> 21
   <212> DNA
   <213> Artificial Sequence
<220>
   <223> oligonucleotide primer
<400> 8
   ggaggtctat ataagcagag c 21
<210> 9
   <211> 20
   <212> DNA
   <213> Artificial Sequence
<220>
   <223> oligonucleotide primer
<400> 9
   tgagccctcg ccccagtcag 20
<210> 10
   <211> 25
   <212> DNA
   <213> Artificial Sequence
<220>
   <223> oligonucleotide primer
<400> 10
   acatacagga'aagccttgcc caaaa 25
<210> 11
   <211> 25
   <212> DNA
   <213> Artificial Sequence
<220>
   <223> oligonucleotide primer
<400> 11
   aaactaccct gcgattctct gctgc 25
<210> 12
   <211> 21
   <212> DNA
   <213> Artificial Sequence
<220>
   <223> oligonucleotide primer
<400> 12
   ggtaaatgga gcttggctga g 21
<210> 13
   <211> 25
   <212> DNA
   <213> Artificial Sequence
<220>
   <223> oligonucleotide primer
<400> 13
   tctggacgtc ctcctgctgg tatag 25
<210> 14
   <211> 25
   <212> DNA
   <213> Artificial Sequence
<220>
   <223> oligonucleotide primer
<400> 14
   ggtatggagc caggggcaag ttggg 25
<210> 15
   <211> 27
   <212> DNA
   <213> Artificial Sequence
<220>
   <223> oligonucleotide primer
<400> 15
   gagtggcaac ttccagggcc aggagag 27
<210> 16
   <211> 27
   <212> DNA
   <213> Artificial Sequence
<220>
   <223> oligonucleotide primer
<400> 16
   cttttgctag cctcaaccct gactatc 27
<210> 17
   <211> 35
   <212> DNA
   <213> Artificial Sequence
<220>
   <223> oligonucleotide primer ZC20,180
<400> 17
   cgcgcggttt aaacgccacc atgagcctct tcggg 35
<210> 18
   <211> 32
   <212> DNA
   <213> Artificial Sequence
<220>
   <223> oligonucleotide primer ZC20,181
<400> 18
   cgtatcggcg cgccctatcc tcctgtgctc cc 32